(19) Europäisches Patentamt
European Patent Office
Office européen des brevets

(11) EP 0 765 878 A1

(12) EUROPÄISCHE PATENTANMELDUNG

(43) Veröffentlichungstag: **02.04.1997 Patentblatt 1997/14**

(51) Int. Cl.[6]: **C07D 409/12**, C07D 401/12, C07D 215/14, C07D 213/65, A61K 31/44, A61K 31/47

(21) Anmeldenummer: **96114868.1**

(22) Anmeldetag: **17.09.1996**

(84) Benannte Vertragsstaaten:
**AT BE CH DE DK ES FI FR GB GR IE IT LI LU MC NL PT SE**

(30) Priorität: **29.09.1995 DE 19536378**

(71) Anmelder: **BAYER AG
51368 Leverkusen (DE)**

(72) Erfinder:
- **Connell, Richard, Dr.
Trumbull, CT 0611 (US)**
- **Goldmann, Siegfried, Dr.
42327 Wuppertal (DE)**
- **Müller, Ulrich, Dr.
42111 Wuppertal (DE)**
- **Beuck, Martin, Dr.
40699 Erkrath (DE)**
- **Bischoff, Hilmar, Dr.
42113 Wuppertal (DE)**
- **Denzer, Dirk, Dr.
42115 Wuppertal (DE)**
- **Grützmann, Rudi, Dr.
42657 Solingen (DE)**
- **Wohlfeil, Stefan, Dr.
40721 Hilden (DE)**

(54) **Heterocyclische Aryl-, Alkyl- und Cycloalkylessigsäureamide als Arzneimittel**

(57) Heterocyclische Aryl-, Alkyl- und Cycloalkylessigsäureamide der allgemeinen Formel (I)

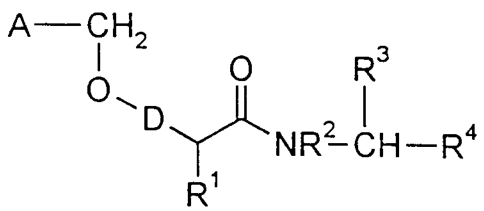

(I)

in welcher A,D,$R^1$,$R^2$,$R^3$ und $R^4$ die in der Beschreibung angegebenen Bedeutungen haben.

Die heterocyclischen Aryl-, Alkyl- und Cycloalkylessigsäureamide werden hergestellt indem man die entsprechend substituierten Essigsäuren mit Aminen, gegebenenfalls in komplexierter Form, umsetzt. Die heterocyclischen Aryl-, Alkyl- und Cycloalkylessigsäureamide können als Wirkstoffe in Arzneimitteln, insbesondere in antiatherosklerotischen Arzneimitteln, verwendet werden.

EP 0 765 878 A1

## Beschreibung

Die vorliegende Erfindung betrifft heterocyclische Aryl-, Alkyl- und Cycloalkylessigsäureamide, ein Verfahren zu ihrer Herstellung und ihre Verwendung als Arzneimittel, insbesondere als antiatherosklerotische Arzneimittel.

Es ist bekannt, daß erhöhte Blutspiegel von Triglyceriden (Hypertriglyceridämie) und Cholesterin (Hypercholesterinämie) mit der Genese von atherosklerotischen Gefäßwand-Veränderungen und koronaren Herzkrankheiten assoziiert sind.

Ein deutlich erhöhtes Risiko für die Entwicklung koronarer Herzerkrankungen liegt darüberhinaus vor, wenn diese beiden Risikofaktoren kombiniert auftreten, was wiederum mit einer Überproduktion an Apoliprotein B-100 einhergeht. Es ist daher nach wie vor ein starkes Bedürfnis, wirksame Arzneimittel zur Bekämpfung der Atherosklerose sowie koronarer Herzkrankheiten zur Verfügung zu stellen.

Die vorliegende Erfindung betrifft heterocyclische Aryl-, Alkyl- und Cycloalkylessigsäurederivate der allgemeinen Formel (I)

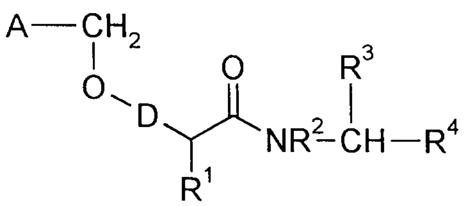 (I)

in welcher

A für Aryl mit 6 bis 10 Kohlenstoffatomen steht, oder für einen 5- bis 7-gliedrigen aromatischen, gegebenenfalls benzokondensierten Heterocyclus mit bis zu 3 Heteroatomen aus der Reihe S, N und/oder O steht, wobei die Ringe gegebenenfalls bis zu 3-fach gleich oder verschieden durch Halogen, Nitro, Cyano, Trifluormethyl, Hydroxy, Carboxyl oder durch geradkettiges oder verzweigtes Alkyl, Alkoxy, Acyl oder Alkoxycarbonyl mit jeweils bis zu 8 Kohlenstoffatomen substituiert sind,

D für Naphthyl, Cycloalkyl mit 3 bis 6 Kohlenstoffatomen oder für einen 4-bis 6-gliedrigen, gesättigten oder ungesättigten, gegebenenfalls benzokondensierten Heterocyclus mit bis zu 3 Heteroatomen aus der Reihe S, N und/oder O steht, wobei die Cyclen gegebenenfalls bis zu 2-fach gleich oder verschieden durch Halogen, Cyano, Hydroxy oder durch geradkettiges oder verzweigtes Alkyl oder Alkoxy mit jeweils bis zu 4 Kohlenstoffatomen substituiert sind,

$R^1$ für Wasserstoff, Cycloalkyl mit 3 bis 10 Kohlenstoffatomen oder für geradkettiges oder verzweigtes Alkyl mit 1 bis 10 Kohlenstoffatomen steht, oder
für Phenyl steht, das gegebenenfalls bis zu 2-fach gleich oder verschieden durch Halogen, Cyano, Hydroxy, geradkettiges oder verzweigtes Alkyl oder Alkoxy mit jeweils bis zu 4 Kohlenstoffatomen substituiert ist,

$R^2$ für Wasserstoff oder geradkettiges oder verzweigtes Alkyl mit bis zu 3 Kohlenstoffatomen steht,

$R^3$ für Wasserstoff oder geradkettiges oder verzweigtes Alkyl mit bis zu 5 Kohlenstoffatomen oder Benzyl steht, oder
für Cycloalkyl mit 3 bis 7 Kohlenstoffatomen steht, oder
für Phenyl oder für einen 5- bis 7-gliedrigen aromatischen Heterocyclus mit bis zu 3 Heteroatomen aus der Reihe S, N und/oder O steht, die gegebenenfalls bis zu 3-fach gleich oder verschieden durch Halogen, Nitro, Phenyl, Hydroxy oder durch geradkettiges oder verzweigtes Alkyl oder Alkoxy mit bis zu 6 Kohlenstoffatomen substituiert sind,

und

$R^4$ für Wasserstoff oder für eine Gruppe der Formel $-CH_2-OH$ oder $CH_2O-CO-R^5$ steht,
worin

$R^5$ Wasserstoff, geradkettiges oder verzweigtes Alkyl mit bis zu 8 Kohlenstoffatomen oder Phenyl bedeutet, das gegebenenfalls bis zu 3-fach gleich oder verschieden durch Halogen, Hydroxy, Cyano oder geradkettiges oder verzweigtes Alkyl oder Alkoxy mit jeweils bis zu 4 Kohlenstoffatomen substituiert ist,

und deren Salze.

Die erfindungsgemäßen heterocyclischen Aryl-, Alkyl- und Cycloalkylessigsäureamide können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium-oder Calciumsalze, sowie Ammoniumsalze, die abgeleitet sind von Ammoniak, oder organischen Aminen, wie beispielsweise Ethylamin, Di- bzw. Triethylamin, Di-bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweiligen Mischungen. Diese Mischungen der Enantiomeren und Diastereomeren lassen sich in bekannter Weise in die stereoisomer einheitlichen Bestandteile trennen.

Heterocyclus, gegebenenfalls benzokondensiert, steht im Rahmen der Erfindung im allgemeinen für einen gesättigten oder ungesättigten 4- bis 7-gliedrigen, vorzugsweise 5- bis 6-gliedrigen Heterocyclus der bis zu 3 Heteroatome aus der Reihe S, N und/oder O enthalten kann und der im Fall eines Stickstoffatoms auch über dieses gebunden sein kann. Beispielsweise seien genannt: Indolyl, Chinolyl, Benzo[b]thienyl, Benzo[b]furyl, Pyridyl, Thienyl, Furyl, Pyrrolyl, Thiazolyl, Oxazolyl, Imidazolyl, Morpholinyl oder Piperidyl. Bevorzugt sind Chinolyl, Furyl, Pyridyl und Thienyl.

Bevorzugt sind Verbindungen der allgemeinen Formel (I), in welcher

A für Phenyl, Chinolyl, Indolyl, Isochinolyl, Benzo[b]furyl, Benzo[b]thienyl oder Pyridyl steht, die gegebenenfalls bis zu 2-fach gleich oder verschieden durch Fluor, Chlor, Brom, Nitro, Cyano, Trifluormethyl, Hydroxy, Carboxyl oder durch geradkettiges oder verzweigtes Alkyl, Alkoxy, Acyl oder Alkoxycarbonyl mit jeweils bis zu 6 Kohlenstoffatomen substituiert sind,

D für Naphthyl, Cyclobutyl, Cyclopentyl, Cyclohexyl, Pyridyl, Furyl, Thienyl, Piperidinyl, Pyrimidinyl, Piperazinyl, Pyridazinyl oder Azetidinyl steht, die gegebenenfalls durch Fluor, Chlor, Brom, Cyano, Hydroxy, Methyl oder Methoxy substituiert sind,

$R^1$ für Wasserstoff, Cyclobutyl, Cyclopentyl, Cyclohexyl, Cycloheptyl, Cylcooctyl oder für geradkettiges oder verzweigtes Alkyl mit bis zu 7 Kohlenstoffatomen steht, oder
für Phenyl steht, das gegebenenfalls durch Fluor, Chlor, Brom, Cyano, Hydroxy oder geradkettiges oder verzweigtes Alkyl oder Alkoxy mit jeweils bis zu 3 Kohlenstoffatomen substituiert ist,

$R^2$ für Wasserstoff oder Methyl steht,

$R^3$ für Wasserstoff oder geradkettiges oder verzweigtes Alkyl mit bis zu 4 Kohlenstoffatomen, Benzyl, Cyclopropyl, Cyclopentyl, Cyclohexyl oder für Phenyl, Pyridyl, Thienyl oder Furyl steht, die gegebenenfalls bis zu 2-fach gleich oder verschieden durch Fluor, Chlor, Brom, Phenyl, Nitro, Hydroxy oder durch geradkettiges oder verzweigtes Alkyl oder Alkoxy mit bis zu 4 Kohlenstoffatomen substituiert sind,

und

$R^4$ für Wasserstoff oder für eine Gruppe der Formel $-CH_2-OH$ oder $-CH_2O-CO-R^5$ steht,
worin

$R^5$ Wasserstoff, geradkettiges oder verzweigtes Alkyl mit bis zu 6 Kohlenstoffatomen oder Phenyl bedeutet, das gegebenenfalls bis zu 2-fach gleich oder verschieden durch Fluor, Chlor, Brom, Cyano, Hydroxy oder geradkettiges oder verzweigtes Alkyl oder Alkoxy mit jeweils bis zu 3 Kohlenstoffatomen substituiert ist,

und deren Salze.

Besonders bevorzugt sind Verbindungen der allgemeinen Formel (I),
in welcher

A für Phenyl, Chinolyl oder Pyridyl steht, die gegebenenfalls durch Fluor, Chlor, Brom, Cyano oder durch geradkettiges oder verzweigtes Alkyl, Alkoxy, Acyl oder Alkoxycarbonyl mit jeweils bis zu 3 Kohlenstoffatomen substituiert sind,

D für Naphthyl, Cyclobutyl, Cyclopentyl, Cyclohexyl, Furyl, Pyridyl, Thienyl, Azetidinyl oder Piperidinyl steht, die gegebenenfalls durch Fluor, Cyano, Hydroxy, Methyl oder Methoxy substituiert sind,

$R^1$ für Wasserstoff, Cyclohexyl, Cycloheptyl, Cyclooctyl oder für geradkettiges oder verzweigtes Alkyl mit bis zu 6 Kohlenstoffatomen steht, oder für Phenyl steht, das gegebenenfalls durch Fluor, Chlor, Cyano, Hydroxy, Methyl oder Methoxy substituiert ist,

$R^2$ für Wasserstoff oder Methyl steht,

$R^3$ für Wasserstoff oder geradkettiges oder verzweigtes Alkyl mit bis zu 3 Kohlenstoffatomen, Benzyl, Cyclopropyl, Cyclopentyl, Cyclohexyl oder für Pyridyl, Thienyl oder Phenyl steht, die gegebenenfalls bis zu 2-fach gleich oder verschieden durch Fluor, Chlor, Brom, Nitro, Phenyl, Hydroxy oder durch geradkettiges oder verzweigtes Alkyl oder Alkoxy mit bis zu 3 Kohlenstoffatomen substituiert sind,

und

$R^4$ für Wasserstoff, für eine Gruppe der Formel $-CH_2-OH$ oder $-CH_2O-CO-R^5$ steht, worin

$R^5$ geradkettiges oder verzweigtes Alkyl mit bis zu 6 Kohlenstoffatomen oder Phenyl bedeutet, das gegebenenfalls bis zu 2-fach gleich oder verschieden durch Fluor, Chlor, Brom, Cyano, Hydroxy, Methyl oder Methoxy substituiert ist,

und deren Salze.

Außerdem wurde ein Verfahren zur Herstellung der erfindungsgemäßen Verbindungen der allgemeinen Formel (I) gefunden,
dadurch gekennzeichnet, daß man
Carbonsäuren oder Ester der allgemeinen Formel (II)

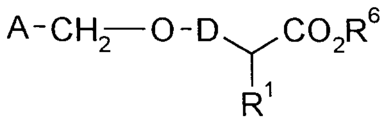

(II)

in welcher

A, D und $R^1$ die oben angegebene Bedeutung haben,

und

$R^6$ für Wasserstoff oder für geradkettiges oder verzweigtes Alkyl mit bis zu 4 Kohlenstoffatomen steht,

mit Aminen der allgemeinen Formel (III)

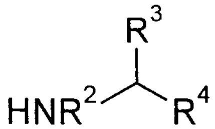

(III)

in welcher

$R^2$, $R^3$ und $R^4$ die oben angegebene Bedeutung haben,

gegebenenfalls in komplexierter Form in inerten Lösemitteln und in Anwesenheit von Basen und/oder Hilfsstoffen umsetzt.

Das erfindungsgemäße Verfahren kann durch folgendes Formelschema beispielhaft erläutert werden:

[A]

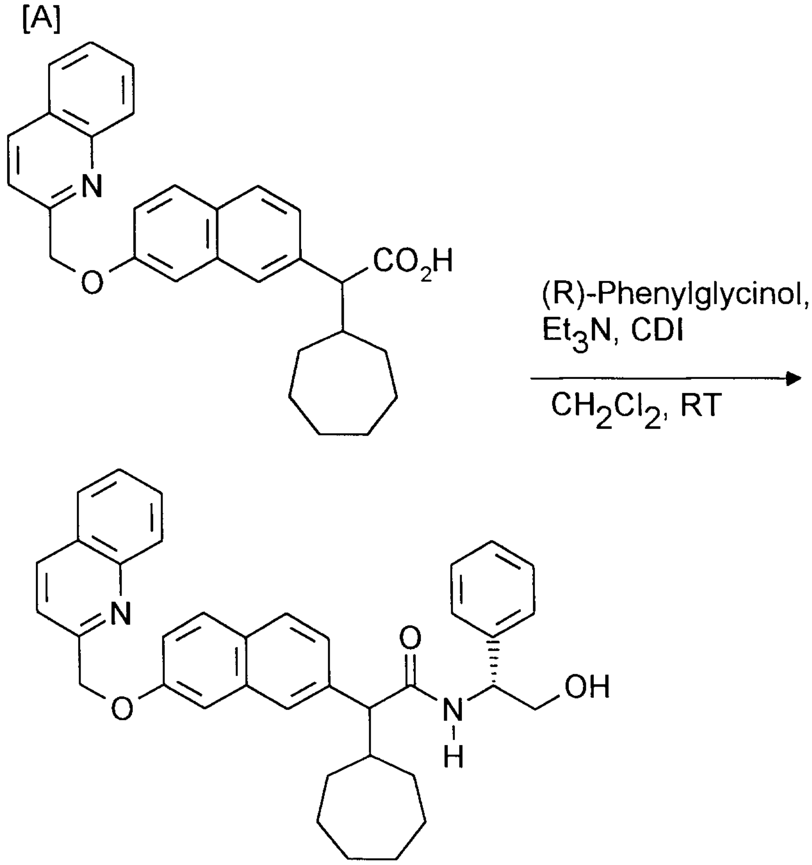


Als Lösemittel eignen sich hierbei inerte organische Lösemittel, die sich unter den Reaktionsbedingungen nicht verändern. Hierzu gehören Ether, wie Diethylether oder Tetrahydrofuran, Halogenkohlenwasserstoffe wie Dichlormethan, Trichlormethan, Tetrachlormethan, 1,2-Dichlorethan, Trichlorethan, Tetrachlorethan, 1,2-Dichlorethylen oder Trichlorethylen, Kohenwasserstoffe wie Benzol, Xylol, Toluol, Hexan, Cyclohexan oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt sind Dichlormethan, Tetrahydrofuran, Toluol und Dimethylformamid.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium-oder Kaliumethanolat oder Kalium-tert.butylat, oder organische Amine (Trialkyl($C_1$-$C_6$)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle wie Natrium und deren Hydride wie Natriumhydrid einzusetzen. Bevorzugt sind Natrium- und Kaliumcarbonat und Triethylamin.

Die Base wird in einer Menge von 1 mol bis 5 mol, bevorzugt von 1 mol bis 3 mol, bezogen auf 1 mol der Verbindung der allgemeinen Formel (II) eingesetzt.

Als Hilfsmittel eignen sich ebenso Dehydratisierungsreagenzien. Dazu gehören beispielsweise Carbodiimide wie Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Iso-butylchloroformat oder Benzotriazolyloxy-tris-(dimethylamino)phosphoniumhexa-fluorophosphat oder Phosphorsäurediphenyl-esteramid oder Methan-sulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die Reaktion wird im allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgeführt werden (z.B. 0,5 bis 5 bar). Im allgemeinen arbeitet man bei Normaldruck.

Als komplexierte Amine eignen sich Metallkomplexe, wobei die metallkomplexbildende Komponente im allgemeinen $C_1$-$C_4$-Aluminium- oder Zinnalkyle, wie beispielsweise $(CH_3)_3Al$ oder $Sn[N(Si(CH_3)_3)_2]_2$ sind. Bevorzugt ist $(CH_3)_3Al$.

Die Verbindungen der allgemeinen Formel (II) sind bekannt oder können beispielsweise hergestellt werden, indem man

[A] Verbindungen der allgemeinen Formel (IV)

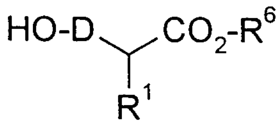

(IV)

in welcher

D, $R^1$ und $R^6$ die oben angegebene Bedeutung haben,

mit Verbindungen der allgemeinen Formel (V)

$$A\text{-}CH_2\text{-}L \qquad (V)$$

in welcher

A die oben angegebene Bedeutung hat

und

L für Sulfonate, wie beispielsweise Mesylat oder Tosylat steht, oder für Halogen, vorzugsweise für Chlor oder Brom steht, in inerten Lösemitteln und in Anwesenheit von Basen und/oder Hilfsmitteln umsetzt,

oder

[B] Verbindungen der allgemeinen Formel (VI)

$$A\text{-}CH_2\text{-}OH \qquad (VI)$$

in welcher

A die oben angegebene Bedeutung hat,

mit Verbindungen der allgemeinen Formel (VII)

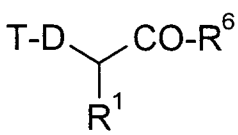

(VII)

in welcher

D, $R^1$ und $R^6$ die oben angegebene Bedeutung haben

und

T für Triflat oder für Halogen, vorzugsweise für Jod oder Brom, steht,

in inerten Lösemitteln, gegebenenfalls in Anwesenheit von Basen und/oder Hilfsmitteln umsetzt, oder

[C] Verbindungen der allgemeinen Formel (VIII)

$$A\text{-}CH_2\text{-}O\text{-}D'\text{-}H \qquad (VIII)$$

in welcher

A die oben angegebene Bedeutung hat

und

D' für den gegebenenfalls substituierten Rest der Formel

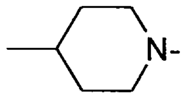 oder 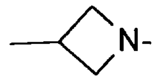

steht,
zunächst durch Umsetzung mit Aldehyden der Formel (IX)

$$R^1\text{-}CHO \qquad (IX)$$

in welcher

$R^1$ die oben angegebene Bedeutung hat,

und Diethylcyanophosphat in inerten Lösemitteln in die Verbindungen der allgemeinen Formel (X)

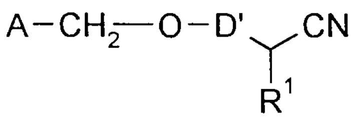 (X)

in welcher

A, D' und $R^1$ die oben angegebene Bedeutung haben,

überführt,
in einem zweiten Schritt mit Alkoholen in Anwesenheit von Säuren in die entsprechenden Ester überführt,
und
im Fall der Säuren die Ester verseift.

Für die Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dimethylformamid, Tetrahydrofuran und Dimethylsulfoxid.

Für das Verfahren eignen sich im allgemeinen Alkohole wie beispielsweise Methanol, Ethanol, Propanol, Isopropanol, Butanol, iso-Butanol oder tert. Butanol. Bevorzugt ist tert.Butanol.

Als Basen für die Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate

wie Calciumcarbonat, oder Alkali- oder Erdalkalialkoholate wie Natrium- oder Kaliummethanolat, Natrium- oder Kaliumethanolat oder Kalium-tert.butylat, oder Alkyllithiumverbindungen, wie beispielsweise Methyl-, sec.Butyl- oder tert.Butyllithium, oder organische Amine (Trialkyl($C_1$-$C_6$)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle wie Natrium oder deren Hydride wie Natriumhydrid einzusetzen. Bevorzugt sind Natriumhydrid, Kaliumcarbonat, Triethylamin, Pyridin und Kalium-tert.butylat, DBU oder DABCO.

Im allgemeinen setzt man die Base in einer Menge von 0,05 mol bis 10 mol, bevorzugt von 1 mol bis 2 mol, bezogen auf 1 mol der Verbindung der Formeln (V) und (VII) ein.

Als Hilfsstoffe für die Umsetzung der Verbindungen der allgemeinen Formel (VII) eignen sich im allgemeinen Kupfer(I)bromid, $CuCO_3$, $Cu(OH)_2$, Cu(I)J, Cu, CuO, CuCl, Cu Bronze, $Cu_2O$ oder Silberoxide. Bevorzugt ist Kupferbromid.

Der Hilfsstoff wird im allgemeinen in einer Menge von 0.01 mol bis 100 mol, bevorzugt von 0.1 mol bis 10 mol, bezogen auf 1 mol der Verbindung der allgemeinen Formel (VII) eingesetzt.

Die erfindungsgemäßen Verfahren werden im allgemeinen in einem Temperaturbereich von -30°C bis +100°C, bevorzugt von -10°C bis +60°C, durchgeführt.

Die erfindungsgemäßen Verfahren werden im allgemeinen bei Normaldruck durchgeführt. Es ist aber auch möglich, die Verfahren bei Überdruck oder bei Unterdruck durchzuführen (z.B. in einem Bereich von 0,5 bis 5 bar).

Als Hilfsmittel eignen sich die oben aufgeführten Dehydratisierungsreagenzien.

Die Herstellung der verschiedenen Amin/Amidderivate kann auch erfolgen unter Anwendung einer Vielzahl von bekannten Methoden, wie beispielsweise die Kopplung von Säurederivaten aus der Peptidchemie [vgl. hierzu Bodanszky, The Practice of Peptide Synthesis: Springer Verlag, Vol 21, 1984 and Organic Chemistry; Functional Group Transformations: Academic Press, Vol 12-1].

Die säurebindenden Mittel und Dehydratisierungsreagenzien werden im allgemeinen in einer Menge von 0,5 bis 3 mol, bevorzugt von 1 bis 1,5 mol, bezogen auf 1 mol der Verbindungen der allgemeinen Formeln (IV) und (VII) eingesetzt.

Die Verseifung der Carbonsäureester erfolgt nach üblichen Methoden, indem man die Ester in inerten Lösemitteln mit üblichen Basen behandelt.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol bezogen auf 1 Mol des Esters eingesetzt. Besonders bevorzugt verwendet man molare Mengen der Reaktanden.

Als Säuren für die Hydrolyse eignen sich im allgemeinen Protonensäuren, wie beispielsweise Schwefelsäure, HCl oder HBr. Bevorzugt ist Schwefelsäure.

Die Säure wird in einer Menge von 0.01 mol bis 200 mol, bevorzugt von 0.1 mol bis 20 mol, jeweils bezogen auf 1 mol der Verbindungen der allgemeinen Formel (VIII) eingesetzt.

Die Verbindungen der allgemeinen Formeln (IV), (V), (VI), (VII), (VIII), (IX) und (X) sind teilweise bekannt oder neu und können in diesen Fällen nach üblichen Methoden hergestellt werden.

Die Verbindungen der allgemeinen Formel (III) sind an sich bekannt.

Die erfindungsgemäßen Verbindungen der allgemeinen Formel (I) haben ein nicht vorhersehbares pharmakologisches Wirkspektrum.

Sie können als Wirkstoffe in Arzneimitteln zur Reduzierung von Veränderungen an Gefäßwänden Verwendung finden und zur Behandlung von Koronaren Herzerkrankungen, Herzinsuffizienz, Störungen der Hirnleistung, ischämischen Gehirnerkrankungen, Apoplexie, Durchblutungsstörungen, Mikrozirkulationsstörungen und Thrombosen.

Weiterhin spielt bei der Okklusion von Gefäßen die Proliferation glatter Muskelzellen eine ausschlaggebende Rolle. Die erfindungsgemäßen Verbindungen sind geeignet, diese Proliferation zu inhibieren und damit atherosklerotische Prozesse zu verhindern.

Die erfindungsgemäßen Verbindungen zeichnen sich durch eine Senkung der ApoB-100-assoziierten Lipoproteinen (VLDL und seiner Abbauprodukte, wie z.B. LDL), des ApoB-100, der Triglyceride und des Cholesterins aus. Damit

besitzen sie wertvolle, im Vergleich zum Stand der Technik überlegene pharmakologische Eigenschaften.

Überraschenderweise besteht die Wirkung der erfindungsgemäßen Verbindungen zunächst in einer Verminderung oder vollständigen Inhibierung der Bildung und/oder der Freisetzung von ApoB-100-assoziierten Lipoproteinen aus Leberzellen, was eine Senkung des VLDL-Plasmaspiegels zur Folge hat. Diese VLDL-Senkung muß mit einer Senkung der Plasmaspiegel von ApoB-100, LDL, Triglyceriden und von Cholesterin einhergehen; es werden also gleichzeitig mehrere der obengenannten Risikofaktoren gesenkt, die an Gefäßwandveränderungen beteiligt sind.

Die erfindungsgemäßen Verbindungen können daher zur Präventation und Behandlung von Atherosklerose, der Fettsucht, Pankreatitis und der Obstipation eingesetzt werden.

**1. Hemmung der Freisetzung ApoB-100-assoziierter Lipoproteine**

Der Test zum Nachweis der Hemmung der Freisetzung ApoB-100-assoziierter Liproproteine aus Leberzellen erfolgte in vitro mit kultivierten Leberzellen, bevorzugt mit Zellen der humanen Linie HepG2. Diese Zellen werden unter Standardbedingungen in Medium für die Kultur eukariontischer Zellen gezüchtet, bevorzugt in RPMI 1640 mit 10% fötalem Kälberserum. HepG2-Zellen synthetisieren und sezernieren in den Kulturüberstand ApoB-100-assoziierte Lipoproteinpartikel, die im Prinzip ähnlich aufgebaut sind wie die VLDL- bzw. LDL-Partikel, die im Plasma zu finden sind.

Diese Partikel können mit einem Immunoassay für humanes LDL nachgewiesen werden. Dieser Immunoassay erfolgt mit Antikörpern, die im Kaninchen gegen humanes LDL unter Standardbedingungen induziert worden waren. Die anti-LDL-Antikörper (Kan-anti-LDL-Ak) wurden an einem Immunosorbens mit humanem LDL affinitätschromatographisch gereinigt. Diese gereinigten Kan-anti-LDL-Ak werden an die Oberfläche von Plastik adsorbiert. Zweckmäßigerweise erfolgt diese Adsorbtion an die Plastikoberfläche von Mikrotitierplatten mit 96 Vertiefungen, bevorzugt an MaxiSorp-Platten. Wenn im Überstand von Hep-G2-Zellen ApoB-100-assoziierte Partikel vorhanden sind, dann können diese an die insolubilisierten Kan-anti-LDL-Ak binden, und es entsteht ein Immunkomplex, der an die Plastikoberfläche gebunden ist. Nicht gebundene Proteine werden durch Waschen entfernt. Der sich an der Plastikoberfläche befindliche Immunkomplex wird mit monoklonalen Antikörpern nachgewiesen, die nach Standardbedingungen gegen humanes LDL induziert und gereinigt worden waren. Diese Antikörper wurden mit dem Enzym Peroxidase konjugiert. Peroxidase setzt das farblose Substrat TMB in Gegenwart von $H_2O_2$ in ein gefärbtes Produkt um. Nach Ansäuerung des Reaktionsgemisches mit $H_2SO_4$ wird die spezifische Lichtabsorption bei 450 nm bestimmt, die ein Maß für die Menge von ApoB-100-assoziierten Partikeln ist, die von den HepG2-Zellen in den Kulturüberstand sezerniert worden waren.

Überraschenderweise hemmen die erfindungsgemäßen Verbindungen die Freisetzung der ApoB-100-assoziierten Partikel. Der $IC_{50}$-Wert gibt an, bei welcher Substanzkonzentration die Lichtabsorption im Vergleich zur Kontrolle (Lösemittelkontrolle ohne Substanz) um 50% inhibiert ist.

| Bsp.-Nr. | $IC_{50}$ [$10^{-9}$ mol/l] |
|---|---|
| 1 | 195 |
| 2 | >2000 |
| 3 | 71 |
| 4 | 11 |
| 5 | 622 |
| 6 | 255 |
| 7 | 1792 |
| 8 | 1883 |
| 9 | >2000 |
| 10 | >2000 |
| 11 | >2000 |
| 12 | >2000 |
| 13 | 235 |
| 14 | 235 |

## 2. Bestimmung der VLDL-Sekretion in vivo am Hamster

Der Effekt der Testsubstanzen auf die VLDL-Sekretion in vivo wird am Hamster untersucht. Hierzu werden Goldhamster nach Prämedikation mit Atropin (83 mg/kg s.c.) mit Ketavet (83 mg/kg s.c.) und Nembutal (50 mg/kg i.p.) narkotisiert. Wenn die Tiere reflexfrei geworden sind, wird die V. jugularis freipräpariert und kanüliert. Anschließend werden 0,25 ml/kg einer 20%igen Lösung von Triton WR-1339 in physiologischer Kochsalzlösung appliziert. Dieses Detergens hemmt die Lipoproteinlipase und führt so zu einem Anstieg des Triglyceridspiegels aufgrund eines ausbleibenden Katabolismus von sezernierten VLDL-Partikeln. Dieser Triglyceridanstieg kann als Maß für die VLDL-Sekretionsrate herangezogen werden. Den Tieren wird vor sowie ein und zwei Stunden nach Applikation des Detergens durch Punktion des retroorbitalen Venenplexus Blut entnommen. Das Blut wird zwei Stunden bei Raumtemperatur, anschließend über Nacht bei 4°C inkubiert, um die Gerinnung vollständig abzuschließen. Danach wird 5 Minuten bei 10.000 g zentrifugiert. Im so erhaltenen Serum wird die Triglyceridkonzentration mit Hilfe eines modifizierten kommerziell erhältlichen Enzymtests bestimmt (Merckotest® Triglyceride Nr. 14354). 100 µl Serum werden mit 100 µl Testreagenz in 96-Lochplatten versetzt und 10 Minuten bei Raumtemperatur inkubiert. Anschließend wird die optische Dichte bei einer Wellenlänge von 492 nm in einem automatischen Platten-Lesegerät bestimmt (SLT-Spectra). Serumproben mit einer zu hohen Triglyceridkonzentration werden mit physiologischer Kochsalzlösung verdünnt. Die in den Proben enthaltene Triglyceridkonzentration wird mit Hilfe einer parallel gemessenen Standardkurve bestimmt. Testsubstanzen werden in diesem Modell entweder unmittelbar vor Applikation des Detergens intravenös verabreicht oder vor Einleitung der Narkose oral oder subkutan.

## 3. Hemmung der intestinalen Triglyceridabsoprtion in vivo (Ratten)

Die Substanzen, die auf ihre triglyceridabsorptionshemmende Wirkung in vivo untersucht werden sollen, werden männlichen Wistar-Ratten mit einem Körpergewicht zwischen 170 und 230 g oral verabreicht. Zu diesem Zweck werden die Tiere 18 Stunden vor der Substanzapplikation in Gruppen zu 6 Tieren eingeteilt und anschließend wird ihnen das Futter entzogen. Trinkwasser steht den Tieren ad libitum zur Verfügung. Die Tiere der Kontrollgruppen erhalten eine wäßrige Traganth-Suspension bzw. eine Traganth-Suspension die Olivenöl enthält. Die Traganth-Olivenöl-Suspension wird mit dem Ultra-Turrax hergestellt. Die zu untersuchenden Substanzen werden in einer entsprechenden Traganth-Olivenöl-Suspension ebenfalls mit dem Ultra-Turrax, direkt vor der Substanzapplikation suspendiert.

Jeder Ratte wird vor der Schlundsondenapplikation zur Bestimmung des basalen Serumtriglyceridgehaltes Blut durch Punktion des retroorbitalen Venenplexus entnommen. Anschließend werden die Traganth-Suspension, die Traganth-Olivenöl-Suspensionen ohne Substanz (Kontrolltiere), bzw. die Substanzen, suspendiert in einer entsprechenden Traganth-Olivenöl-Suspension, den nüchternen Tieren mit einer Schlundsonde verabreicht. Die weiteren Blutentnahmen zur Bestimmung des postprandialen Serumtriglyceridanstiegs erfolgen in der Regel 1, 2 und 3 Stunden nach der Schlundsondenapplikation.

Die Blutproben werden zentrifugiert und nach Gewinnung des Serums die Triglyceride photometrisch mit einem EPOS-Analyzer 5060 (Eppendorf Gerätebau, Netheler & Hinz GmbH, Hamburg) bestimmt. Die Bestimmung der Trigylceride erfolgt vollenzymatisch mit einem handelsüblichen UV-Test.

Der postprandiale Serumtriglyceridanstieg wird durch Subtraktion des Triglyceridvorwertes jeden Tieres von seinen korrespondierenden postprandialen Triglyceridkonzentrationen (1, 2 und 3 Stunden nach Applikation) ermittelt.

Die Differenzen (in mmol/l) zu jedem Zeitpunkt (1, 2 und 3 Stunden) werden in den Gruppen gemittelt, und die Mittelwerte des Serumtriglyceridanstiegs (ΔTG) der substanzbehandelten Tiere mit den Tieren verglichen, die nur die Traganth-Öl-Suspension erhielten.

Ebenso wird der Serumtriglyceridverlauf der Kontrolltiere, die nur Traganth erhielten, berechnet. Der Substanzeffekt zu jedem Zeitpunkt (1, 2 oder 3 Stunden) wird wie folgt ermittelt und in Δ% von der ölbelasteten Kontrolle angegeben.

$$\Delta\%\ \text{Triglyceridanstieg} = \frac{\Delta TG_{\text{Substanz}} - \Delta TG_{\text{Traganthkontrolle}}}{\Delta TG_{\text{Ölbelastung}} - \Delta TG_{\text{Traganthkontrolle}}} \times 100$$

Effekt von 10 mg Prüfsubstanz / kg KG p.o. auf den Triglyceridanstieg (Δ%) 2 h nach einer Triglyceridbelastung im Serum nüchterner Ratten. Der Serumtriglyceridanstieg fettbelasteter Kontrolltiere bezogen auf den Serumtriglyceridspiegel von Traganth-Kontrolltieren entspricht 100%. n = 6 Tiere pro Gruppe.

Die statistische Auswertung erfolgt mit Student's t-Test nach vorheriger Überprüfung der Varianzen auf Homogenität.

Substanzen, die zu einem Zeitpunkt den postprandialen Serumtriglyceridanstieg, verglichen mit dem der unbehandelten Kontrollgruppe, statistisch signifikant (p <0,05) um mindestens 30 % vermindern, werden als pharmakologisch wirksam angesehen.

### 4. Hemmung der VLDL-Sekretion in vivo (Ratte)

Die Wirkung der Testsubstanzen auf die VLDL-Sekretion wird ebenfalls an der Ratte untersucht. Dazu wird Ratten 500 mg/kg Körpergewicht Triton WR-1339 (2,5 mg/kg), gelöst in physiologischer Kochsalzlösung, intravenös in die Schwanzvene appliziert. Triton WR-1339 inhibiert die Lipoproteinlipase und führt somit durch Hemmung des VLDL-Katabolismus zu einem Anstieg des Triglycerid- und Cholsterinspiegels. Diese Anstiege können als Maß für die VLDL-Sekretionsrate herangezogen werden.

Den Tieren wird vor sowie eine und zwei Stunden nach Applikation des Detergens durch Punktion des retroorbitalen Venenplexus Blut entnommen Das Blut wird zur Gerinnung 1 h bei Raumtemperatur inkubiert und das Serum durch Zentrifugation mit 10 000 g für 20 s gewonnen. Anschließend werden die Triglyceride mittels eines handelsüblichen gekoppelten Enzymtests (Sigma Diagnostics®, Nr. 339) bei einer Wellenlänge von 540 nm photometrisch bestimmt. Die Messung erfolgt mit Hilfe eines ebenfalls gekoppelten Enzymtests (Boehringer Mannheim®, Nr. 1442350) bei einer Wellenlänge von 546 nm. Proben mit Triglycerid- bzw. Cholesterinkonzentrationen, die den Meßbereich der Methoden überschreiten, werden mit physiologischer Kochsalzlösung verdünnt. Die Ermittlung der jeweiligen Serumkonzentrationen erfolgt anhand parallel gemessener Standardreihen. Testsubstanzen werden unmittelbar nach der Tritoninjektion oral, intravenös oder subcutan appliziert.

Die Erfindung betrifft außerdem die Kombination von heterocyclischen Aryl-, Alkyl- und Cycloalkylessigsäureamiden der allgemeinen Formel (I) mit einem Glucosidase- und/oder Amylasehemmer zur Behandlung von familiärer Hyperlipidamien, der Fettsucht (Adipositas) und des Diabetes mellitus. Glucosidase- und/oder Amylasehemmer im Rahmen der Erfindung sind beispielsweise Acarbose, Adiposine, Voglibase, Miglitol, Emiglitate, MDL-25637, Camiglibase (MDL-73945), Tendamistate, AI-3688, Trestatin, Pradimilin-Q und Salbostatin.

Bevorzugt ist die Kombination von Acarbose, Miglitol, Emiglitate oder Voglibase mit einer der oben aufgeführten erfindungsgemäßen Verbindungen der allgemeinen Formel (I).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

**Verwendete Abkürzungen:**

| | |
|---|---|
| bs | = broad singlet |
| CI | = Chemische Ionisation |
| cHept | = cyclo-Heptyl |
| cHex | = cyclo-Hexyl |
| cPent | = cyclo-Pentyl |
| CDI | = N'-(3-Dimethylaminopropyl)-N'-ethylcarbodiimid |
| d | = doublet |
| dia | = Diastereomer |
| dd | = doublet of doublets |
| DMF | = N,N-Dimethylformamid |
| DMSO | = Dimethylsulfoxid |

| | |
|---|---|
| EI | = Elektronenstoß-Ionisation |
| FAB | = Fast Atom Bombardment |
| HOBT | = 1-Hydroxy-1H-benzotriazol |
| Hz | = Hertz |
| iBu | = iso-Butyl |
| iPr | = iso-Propyl |
| m | = multiplet |
| Me | = Methyl |
| Ph | = Phenyl |
| RT | = Raumtemperatur |
| s | = singlet |
| t | = triplet |
| TFA | = Trifluoressigsäure |
| THF | = Tetrahydrofuran |
| TMS | = Tetramethylsilan |

**Benutzte Solvenzgemische**

| | |
|---|---|
| Petrolether : Aceton | = 1:1 (A) |
| Petrolether : Essigester | = 10:1 (B) |
| Petrolether : Essigester | = 5:1 (C) |
| Petrolether : Essigester | = 2:1 (D) |
| Petrolether : Essigester | = 1:1 (E) |
| Petrolether : Essigester | = 2:1 (F) |
| Dichlormethan | (G) |
| Dichlormethan : Methanol | = 50:1 (H) |
| Dichlormethan : Methanol | = 20:1 (I) |
| Dichlormethan : Methanol | = 10:1 (J) |
| Dichlormethan : Essigester | = 1:1 (K) |
| Dichlormethan : Ethanol | = 50:1 (L) |
| Petrolether : Essigester | = 20:1 (M) |
| Petrolether : Essigester | = 3:1 (N) |
| Toluol : Essigester | = 1:1 (O) |
| Essigester : Methanol | = 10:1 (P) |

**Ausgangsverbindungen**

**Beispiel I**

2-Cycloheptyl-2-(7-methoxy-3,4-dihydro-naphth-2-yl)essigsäureethylester

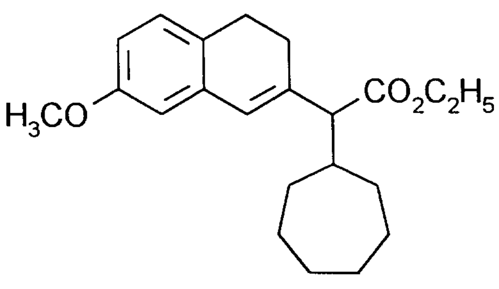

Natriumhydrid (0.59 g, 14.7 mmol, 60%ig in Paraffin) wird in wasserfreiem DMF (1.5 ml) unter Argon vorgelegt. Es wird auf 0 °C abgekühlt. 2-(7-Methoxy-3,4-dihydro-naphthalen-2-yl)essigsäureethylester (3.3 g, 13.4 mmol) und Cycloheptylbromid (2.61 g, 14.7 mmol) werden in DMF (5 ml) gelöst und zugetropft. Es wird 1 Stunde bei 0 °C, und über Nacht unter allmählichen Erwärmung auf Raumtemperatur nachgerührt. Es wird auf Wasser und $CH_2Cl_2$ gegossen, und die abgetrennte wäßrige Phase wird noch 3 mal mit $CH_2Cl_2$ extrahiert. Die vereinigten organischen Phasen werden mit Natriumsulfat getrocknet und eingedampft. Der Rückstand wird säulenchromatographisch gereinigt: Ausbeute:

2.60 g (57%);
$R_f$ = 0.67 ($CH_2Cl_2$);
Masse (berechnet) für $C_{22}H_{30}O_3$ = 342.48; Massenspektrum (CI($NH_3$), rel. Intensität) 360 ($M+NH_4^+$, 100%), 343 (18%);
$^1$H NMR (250 MHz, $CDCl_3$ / TMS) δ 7.00 (d, J = 8.08 Hz, 1 H), 6.65 (dd, J = 8.11 Hz, J = 2.68 Hz, 1 H), 6.60 (d, J = 2.59 Hz, 1 H), 6.34 (s, 1 H), 4.15 (m, 2 H), 3.77 (s, 3 H), 2.95 (d, J = 10.9 Hz, 1 H), 2.71 (m, 2 H), 2.37 (m, 1 H), 2.28 (m, 1 H), 2.10 (m, 1 H), 1.72 - 1.14 (m, 12 H), 1.23 (t, J = 7.08 Hz, 3 H).

**Beispiel II**

2-Cycloheptyl-2-(7-methoxy-naphth-2-yl)essigsäureethylester

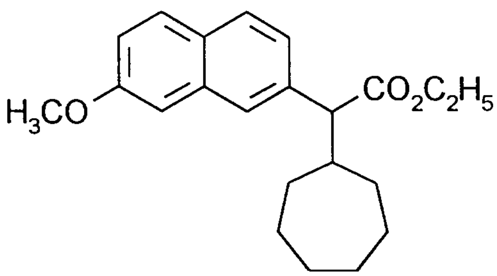

2.60 g (7.6 mmol) der Verbindung aus Beispiel I und Tetrachlor-p-benzochinon (2.80 g, 11.4 mmol) werden in Xylol (20 ml) gelöst und 8 Stunden zum Rückfluß erhitzt. Es wird abgekühlt und das Xylol wird im Rotationsverdampfer abgedampft. Man gibt zu dem Rückstand $CH_2Cl_2$ und die ausgefallenen Kristalle werden abgesaugt und getrocknet. Die Mutterlauge wird einrotiert und mit den Kristallen zusammen säulenchromatographisch gereinigt:
Ausbeute: 1.56 g (60%);
$R_f$ = 0.40 (Petrolether : Essigester 10:1)
Masse (berechnet) für $C_{22}H_{28}O_3$ = 340.47; Massenspektrum (CI($NH_3$), rel. Intensität) 358 ($M+NH_4^+$, 100%);
$^1$H NMR (250 MHz, $CDCl_3$ / TMS) δ 7.75 - 7.62 (m, 3 H), 7.35 (dd, J = 8.47 Hz, J = 1.95 Hz, 1 H), 7.15 - 7.05 (m, 2 H), 4.20 - 3.99 (m, 2 H), 3.91 (s, 3 H), 3.42 (d, J = 10.98 Hz, 1 H), 2.35 (m, 1 H), 1.90 - 1.10 (m, 11 H), 1.20 (t, J = 7.12 Hz, 3 H), 1.05 (m, 1 H).

**Beispiel III**

2-Cycloheptyl-2-(7-hydroxy-naphth-2-yl)essigsäureethylester

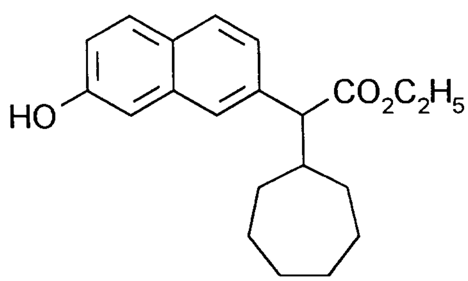

0.5 g (1.47 mmol) der Verbindung aus Beispiel II werden in $CH_2Cl_2$ (10 ml) gelöst. Die Lösung wird auf -78°C gekühlt, und mit Bortribromid (1 M Lösung im $CH_2Cl_2$, 7.34 ml, 7.34 mmol) versetzt. Die Lösung wird auf -30°C und nach einer Stunde auf 0°C erwärmt. Nach einer Stunde wird die Lösung wieder auf -30°C gekühlt und mit Ethanol (10 ml) versetzt. Man läßt über Nacht bei Raumtemperatur rühren. Es wird einrotiert und der Rückstand wird mit Wasser und Essigester verdünnt. Die Phasen werden getrennt und, die organische Phase über Natriumsulfat getrocknet und einrotiert. Der Rückstand wird säulenchromatographisch gereinigt:
Ausbeute: 0.358 g (75%);
Fp. = 106°C;
$R_f$ = 0.43 (3:1; Petrolether : Essigester);

Masse (berechnet) für $C_{21}H_{26}O_3$ = 326.44; Massenspektrum (CI($NH_3$), rel. Intensität) 344 ($M+NH_4^+$, 100%);
$^1$H NMR (300 MHz, $CDCl_3$ / TMS) δ 7.70 (d, J = 8.68 Hz, 2 H), 7.59 (bs, 1 H), 7.35 (dd, J = 8.50 Hz, J = 1.68 Hz, 1 H), 7.10 (d, J = 1.46 Hz, 1 H), 7.05 (dd, J = 8.73 Hz, J = 2.51 Hz, 1 H), 4.10 (m, 2 H), 3.41 (d, J = 10.96 Hz, 1 H), 2.37 (m, 1 H), 1.70 - 1.20 (m, 11 H), 1.19 (t, J = 7.20 Hz, 3 H), 1.00 (m, 1 H).

**Beispiel IV**

1,1-Dimethoxy-2-iodo-1-(6-methoxynaph-2-yl)ethan

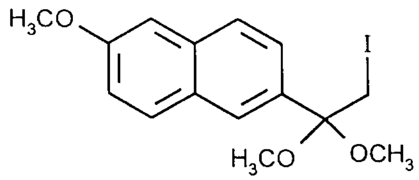

2-Acetyl-6-methoxynaphthalin (18.8 g, 93.9 mmol) wird in Trimethylorthoformiat (76.0 g, 93.4 mmol) in einem dunklen Kolben vorgelegt. Dazu wird Iod (28.6 g, 112.7 mmol) zugegeben. Es wird über Nacht bei Raumtemperatur nachgerührt. Man gibt dann gesättigte wäßrige $Na_2S_2O_3$ hinzu, diese Lösung wird mit $CH_2Cl_2$ (3x) extrahiert. Die vereinigten organischen Phasen werden vereinigt und über $Na_2SO_4$ getrocknet und einrotiert. Der Rückstand wird Flashchromatographiert: Ausbeute: 19.5 g (56%);
Fp. = 95-96°C;
$R_f$ = 0.33 (20:1; Petrolether : Essigester);
$^1$H NMR (200 MHz, $CDCl_3$ / TMS) δ 7.94 (m, 1 H), 7.76 - 7.71 (m, 2 H), 7.46 (dd, J = 1.82 Hz, 1 H), 7.17 - 7.14 (m, 2 H), 3.92 (s, 3 H), 3.59 (s, 2 H), 3.25 (s, 6 H).

**Beispiel V**

2-(6-methoxynaphth-2-yl)essigsauremethylester

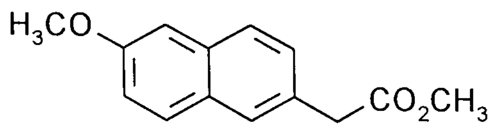

4.1 g (11.01 mmol) der Verbindung aus Beispiel IV werden in Trimethylorthoformiat (11.0 g, 103 mmol) gelöst. Dazu gibt man Methanol (11.0 ml) und unter Lichtausschluß $AgBF_4$ (2.14 g, 11.01 mmol). Es wird über Nacht bei Raumtemperatur nachgerührt. Man gibt dann Wasser und $CH_2Cl_2$ dazu und saugt die unlöslichen Silbersalze ab. Es wird dann extrahiert. Die vereinigten organischen Phasen werden über $Na_2SO_4$ getrocknet und einrotiert. Der Rückstand wird Flashchromatographiert:
Ausbeute: 1.8 g (71%);
Fp. = 70°C;
$R_f$ = 0.14 (20:1; Petrolether : Essigester);
Masse (berechnet) für $C_{14}H_{14}O_3$ = 230.27; Massenspektrum (FAB, rel. Intensität) 230 (70%), 171 (100%);
$^1$H NMR (300 MHz, $CDCl_3$) δ 7.71 - 7.65 (m, 3 H), 7.36 (dd, J= 8.44 Hz, J = 1.81 Hz, 1 H), 7.15 - 7.11 (m, 2 H), 3.91 (s, 3 H), 3.75 (s, 2 H), 3.70 (s, 3 H).

**Beispiel VI**

2-Cyclopentyl-2-(6-methoxynaphth-2-yl)essigsäuremethylester

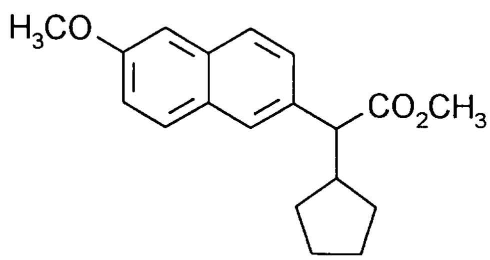

Kaliumtertiärbutylat (4.52 g, 40.2 mmol) wird in DMF (10.0 ml) gelöst. Die Lösung wird anschließend auf 0°C abgekühlt. 6.45 g (28.0 mmol) der Verbindung aus Beispiel V wird in DMF (30.0 ml) gelöst und zugetropft. Man rührt 30 Minuten bei 0°C nach und tropft eine Lösung aus Cyclopentylbromid (6.53 g, 43.7 mmol) in DMF (5.0 ml) hinzu. Die Temperatur steigt allmählich auf Raumtemperatur und läßt über Nacht nachrühren. Es wird $H_2O$ zugegeben und der entstandene Niederschlag wird abgesaugt und getrocknet. Die Kristalle werden mit Methanol umkristallisiert:
Ausbeute: 5.43 g (65%);
Fp. = 115°C
$R_f$ = 0.25 (20:1; Petrolether : Essigester);
Masse (berechnet) für $C_{19}H_{22}O_3$ = 298.38; Massenspektrum (EI, rel. Intensität) 298 (65%), 229 (100%), 171 (100%);
$^1H$ NMR (200 MHz, $CDCl_3$ / TMS) δ 7.78 - 7.67 (m, 3 H), 7.45 (dd, J = 8.55 Hz, J = 1.58 Hz, 1 H), 7.16 - 7.11 (m, 2 H), 3.91 (s, 3 H), 3.65 (s, 3 H), 3.41 (d, J = 11.13 Hz, 1 H), 2.65 (m, 1 H), 1.95 (m, 1 H), 1.75 - 1.20 (m, 6 H), 1.05 (m, 1 H).

**Beispiel VII**

2-Cyclopentyl-2-(6-hydroxynaphth-2-yl)essigsäuremethylester

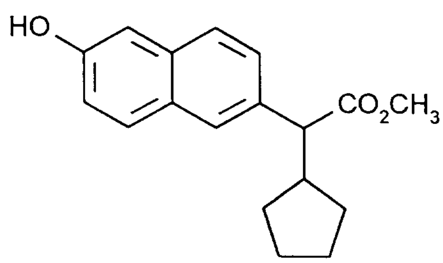

Die Reaktion wird mit 6.83 g (23 mmol) der Verbindung aus Beispiel VI, 1N $BBr_3$ im $CH_2Cl_2$ (100 ml, 100 mmol) analog der Vorschrift für Beispiel III durchgeführt:
Ausbeute: 5.87 g (90%);
$R_f$ = 0.40 (50:1; $CH_2Cl_2$ : Methanol);
Masse (berechnet) für $C_{18}H_{20}O_3$ = 284.36; Massenspektrum (EI, rel. Intensität) 284 (35%), 43 (100%);
$^1H$ NMR (200 MHz, $CDCl_3$/TMS) δ 7.80 - 7.60 (m, 3 H), 7.42 (dd, J = 8.53 Hz, J = 1.77 Hz, 1 H), 7.20 - 7.05 (m, 2H), 3.65 (s, 3 H), 3.39 (d, J = 11.1 Hz, 1 H), 2.70 (m, 1 H), 1.95 (m, 1 H), 1.70 - 1.20 (m, 6 H), 1.03 (m, 1 H).

**Beispiel VIII**

2-(5-Benzyloxy-2-pyridin-2-yl)-2-cycloheptylacetonitril

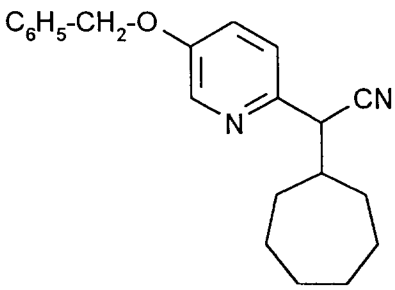

Natriumhydrid (183 mg, 4.58 mmol, 60%ig in Paraffin) wird in wasserfreiem DMF (5 ml) unter Argon vorgelegt. Dazu wird eine Lösung von 5-Benzyloxy-2-cyanomethylpyridin (1.0 g, 4.16 mmol) in DMF (3 ml), bei 0 °C zugetropft. Man läßt auf Raumtemperatur kommen und nach 30 Minuten wird wieder auf 0 °C abgekühlt. Es wird eine Lösung von Cycloheptylbromid (0.81 g, 4.58 mmol) in DMF (3 ml) tropfenweise zugegeben. Es wird 2 Stunden bei Raumtemperatur gerührt. Die Lösung wird mit Wasser versetzt und 3 mal mit Ether extrahiert. Die vereinigten organischen Phasen werden über Natriumsulfat getrocknet und einrotiert. Der Rückstand wird säulenchromatographisch gereinigt:
Ausbeute: 0.83 g (62%);
$R_f$ = 0.53 (3:1; Petrolether : Essigester);
Masse (berechnet) für $C_{21}H_{24}N_2O$ = 320.44; Massenspektrum (CI ($NH_3$), rel. Intensität) 321 (M+H, 100%), 224 (60%), 91 (70%);
$^1$H NMR (250 MHz, TMS/$CDCl_3$) δ 8.34 (m, 1 H), 7.48 - 7.5 (m, 7 H), 5.11 (s, 2 H), 3.87 (d, J = 5.82 Hz, 1 H), 2.20 (m, 1 H), 1.70 - 1.40 (m, 12 H).

**Beispiel IX**

2-(5-Benzyloxy-2-pyridin-2-yl)-2-cyclopentylacetonitril

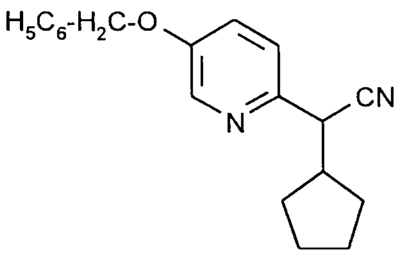

Die Reaktion wird mit 1.0 g (4.16 mmol) 5-Benzyloxy-2-cyanomethylpyridin, Natriumhydrid (0.18 g, 4.58 mmol) und Cyclopentylbromid (0.68 g, 4.58 mmol) analog der Vorschrift für Beispiel VIII durchgeführt:
Ausbeute: 693 mg (57%);
$R_f$ = 0.61 (3:1; Petrolether : Essigester);
Masse (berechnet) für $C_{19}H_{20}N_2O$ = 292.38, Massenspektrum (CI ($NH_3$), rel. Intensität) 293 (M+H, 30%), 224 (30%), 91 (100%);
$^1$H NMR (250 MHz, $CDCl_3$/TMS) δ 8.32 (m, 1 H), 7.45 - 7.32 (m, 5 H), 7.31 - 7.22 (m, 2 H), 5.10 (s, 2 H), 3.90 (d, J = 6.44 Hz, 1 H), 2.48 (m, 1 H), 1.88 - 1.40 (m, 8H).

**Beispiel X**

2-(5-hydroxy-2-pyridin-2-yl)-2-cycloheptylessigsäuremethylester

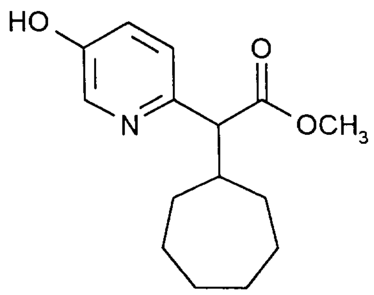

2.0 g (6.24 mmol) der Verbindung aus Beispiel VIII werden in Methanol (8 ml) heiß gelöst. Dazu gibt man konzentrierte Schwefelsäure (3 ml) und läßt alles zusammen 5 Stunden zum Rückfluß kochen. Es wird abgekühlt und mit Essigester (50 ml) verdünnt und vorsichtig mit gesättigter Natriumhydrogencarbonatlösung neutralisiert. Die organische Phase wird über Natriumsulfat getrocknet und eingedampft. Der Rückstand wird säulenchromatographisch gereinigt: Ausbeute: 0.79 g (52%);
Fp. = 141°C
$R_f$ = 0.54 (1:1; Petrolether : Essigester);
Masse (berechnet) für $C_{15}H_{21}NO_3$ = 263.34; Massenspektrum (Cl ($NH_3$), rel. Intensität) 264 (M+H, 100%), 167 (60%), 135 (40%);
$^1$H NMR (200 MHz, $CDCl_3$/TMS) δ 8.09 (d, J = 2.61 Hz, 1 H), 7.40 - 7.23 (m, 2 H), 3.62 (s, 3 H), 3.63 (d, J = 10.71 Hz, 1 H), 2.35 (m, 1 H), 1.80 - 1.15 (m, 11 H), 1.05 (m, 1 H).

**Beispiel XI**

2-(5-Hydroxy-2-pyridin-2-yl)-2-cyclopentylessigsäuremethylester

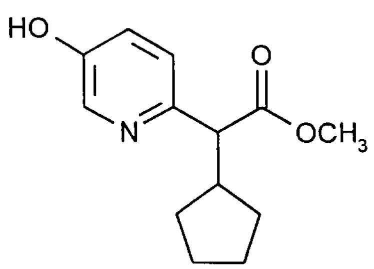

Die Reaktion wird mit 0.732 g (2.5 mmol) der Verbindung aus Beispiel IX, Methanol (5 ml) und konz. Schwefelsäure (1.1 ml) analog der Vorschrift von Beispiel X durchgeführt.
Ausbeute: 0.290 g (49%);
Fp. = 112°C;
$R_f$ (1:1; Petrolether : Essigester) = 0.45;
Masse (berechnet) für $C_{13}H_{17}NO_3$ = 235.29; Massenspektrum (FAB, rel. Intensität) 236 (100%), 176 (60%);
$^1$H NMR (200 MHz, $CDCl_3$/TMS) δ 8.09 (dd, J = 2.78 Hz, J = 0.58 Hz, 1 H), 7.34 (dd, J = 8.60 Hz, J = 0.45 Hz, 1 H), 7.23 (dd, J = 8.59 Hz, J = 2.88 Hz, 1 H), 3.63 (s, 3 H), 3.56 (d, J = 11.10 Hz, 1 H), 2.60 (m, 1 H), 1.90 (m, 1 H), 1.70 - 1.20 (m, 6 H), 1.03 (m, 1 H).

**Beispiel XII**

2-Cycloheptyl-2-(thiophen-2-yl)essigsäureethylester

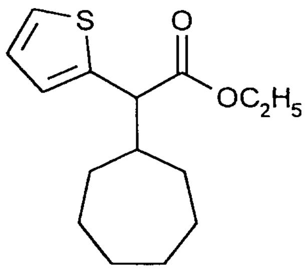

Thienylessigsäureethylester (17.0 g, 100 mmol) werden in wasserfreiem DMF (170 ml) vorgelegt und auf 0°C abgekühlt. Dazu wird portionsweise Natiumhydrid (6.0 g, 150 mmol) zugegeben. Man rührt 30 Minuten nach und tropft anschließend Cycloheptylbromid (53 g, 300 mmol) hinzu. Unter allmählicher Erwärmung auf Raumtemperatur wird über Nacht nachgerührt. Im Hochvakuum wird DMF abrotiert, der Rückstand anschließend in Essigester und gesättigter $NH_4Cl$-Lösung extrahiert. Die organische Phase wird noch 2 mal mit Wasser gewaschen über Natriumsulfat getrocknet und einrotiert. Der Rückstand wird säulenchromatographisch gereinigt:
Ausbeute: 21 g (79%)
$R_f$ = 0.80 (10:1, Petrolether: Essigester);
Masse (berechnet) für $C_{15}H_{22}O_2S$ = 266.40; Massenspektrum (CI ($NH_3$), rel. Intensität) 267 (20%), 170 (100%), 97 (30%);
$^1$H NMR (250 MHz, $CDCl_3$/TMS) $\delta$ 7.18 (m, 1 H), 6.95 - 6.90 (m, 2 H), 4.11 (m, 2 H), 3.62 (d, J = 10.15 Hz, 1 H), 2.15 (m, 1 H), 1.85 - 1.20 (m, 11 H), 1.27 (t, J= 7.1 Hz, 3 H), 1.15 (m, 1 H).

**Beispiel XIII**

2-Cycloheptyl-2-(5-bromothiophen-2-yl)essigsäureethylester

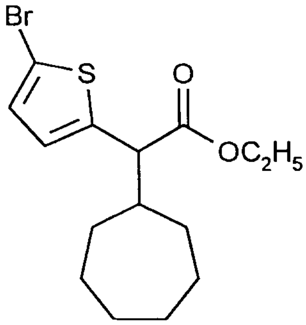

9.37 g (35.2 mmol) der Verbindung aus Beispiel XII werden bei Raumtemperatur in einer 1:1-Mischung aus Chloroform und Essigsäure (42 ml) mit N-Bromsuccinimid (6.89 g, 38.7 mmol) umgesetzt [Literatur: J. Org. Chem., 1968, **33**, 2902]. Nach 2 Stunden ist das Ausgangmaterial verschwunden; man extrahiert mit $CH_2Cl_2$ und wäßriger Natriumhydrogencarbonatlösung, trocknet die organische Phase mit Natriumsulfat und dampft sie im Vakuum ein.
Ausbeute: 12 g (99%)
$R_f$ = 0.73 (Toluol)

**Beispiel XIV**

N-(tert-Butoxycarbonyl)-4-Hydroxypiperidin

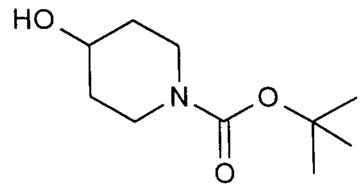

Es wird 4-Hydroxypiperidin (83.7 g, 827.9 mmol) in 800 ml Methanol gelöst und auf 0°C abgekühlt. Eine Lösung aus Di-tert-butyldicarbonat (198.8 g, 910.7 mmol) in 400 ml Methanol werden zugetropft. Man rührt über Nacht bei Raumtemperatur nach. Die Lösung wird einrotiert und der Rückstand wird über Flashsäule gereinigt.
Ausbeute: 157 g (94%);
$R_f$ = 0.36 (1:1; Essigester : Cyclohexan);
Masse (berechnet) für $C_{10}H_{19}NO_3$ = 201.27, Massenspektrum (FAB, rel. Intensität) 201 (5%), 145 (25%), 128 (25%), 57 (100%);
$^1$H NMR (200 MHz, $CDCl_3$) δ 3.88 - 3.80 (m, 3 H, $CH_2\underline{CH}(OH)CH_2$ und $N\underline{CH_2}CH_2$), 3.08 - 2.95 (m, 2 H, $N\underline{CH_2}CH_2$), 1.89 - 1.81 (m, 2 H, $CH(OH)\underline{CH_2}CH_2$), 1.54 - 1.36 (m, 2 H, $CH(OH)\underline{CH_2}CH_2$), 1.45 (s, 9 H, $NCO_2C(\underline{CH_3})_3$).

**Beispiel XV**

4-(Chinolin-2-ylmethoxy)piperidin

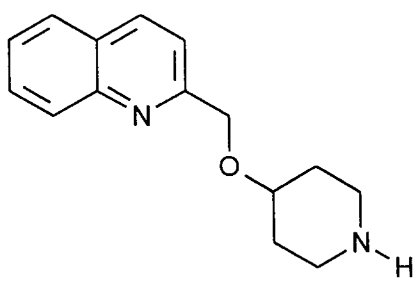

27.5 g (80.3 mmol) N-(tert-butoxycarbonyl)-4-(chinolin-2-ylmethoxy)piperidin (Beispiel XXIII) werden in $CH_2Cl_2$ (250 ml) gelöst. Dazu tropft man Trifluoressigsäure (91.6 g, 803 mmol). Die Mischung wird über Nacht zum Rückfluß gekocht. Es wird abgekühlt und mit Wasser (1000 ml) überschichtet und extrahiert. Die wäßrige Phase wird nochmal mit $CH_2Cl_2$ extrahiert und anschließend abgekühlt. Es wird mit 2 N Natronlauge alkalisch gestellt. Es wird dann 2x mit $CH_2Cl_2$ extrahiert. Die vereinigten organischen Phasen werden über $Na_2SO_4$ getrocknet und einrotiert:
Ausbeute: 14.0 g (72%);
$R_f$ = 0.10 ($CH_2Cl_2$ : Methanol,10 : 1);
Masse (berechnet) für $C_{15}H_{18}N_2O$ = 242.32; Massenspektrum (EI, rel. Intensität) 242 (10%), 158 (50%), 143 (100%), 85 (60%);
$^1$H-NMR (300 MHz, $CDCl_3$) δ 8.17 (d, J = 8.50 Hz, 1 H), 8.05 (d, J = 8.50 Hz, 1 H), 7.81 (dd, J = 8.09 Hz, J = 0.7 Hz, 1 H), 7.60 (m, 2 H), 7.50 (m, 1 H), 4.85 (s, 2 H, $O\underline{CH_2}$-Chinolin), 3.56 (m, 1 H, Chinolin-$CH_2O\underline{CH}(CH_2\text{-})_2$), 3.15 - 3.07 (m, 2 H, $N\underline{CH_2}CH_2$), 2.66 - 2.56 (m, 2 H, $N\underline{CH_2}CH_2$), 2.06 - 1.98 (m, 2 H, $CH(OR)\underline{CH_2}CH_2$), 1.63 - 1.48 (m, 2 H).

**Beispiel XVI**

2-Phenyl-[4-(Chinolin-2-ylmethoxy)-piperidin-1-yl]-acetonitril

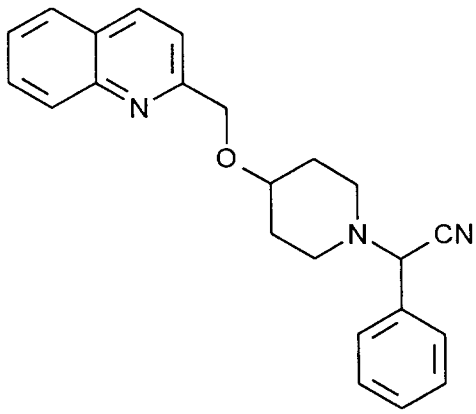

9.8 g (40 mmol) der Verbindung aus Beispiel XV werden in THF (200 ml) suspendiert. Diese wird zu einer Mischung aus Benzaldehyd (5.0 g, 50 mmol) und Diethylcyanophosphat (7.8 g, 48 mmol) in THF (100 ml) zugegeben. Es wird über Nacht bei Raumtemperatur nachgerührt. Das THF wird abrotiert und der Rückstand wird flashchromatographisch gereinigt:
Ausbeute: 13.2 g (91%);
$R_f$ = 0.18 (3:1, Essigester : Petrolether);
Masse (berechnet) für $C_{23}H_{23}N_3O$ = 357.46; Massenspektrum (CI ($NH_3$), rel. Intensität) 358 (70%), 331 (100%);
$^1$H-NMR (200 MHz, $CDCl_3$/TMS) δ 8.17 (d, J = 8.50 Hz, 1 H), 8.04 (d, J = 8.45 Hz, 1 H), 7.81 (bd, J = 8.13 Hz, 1 H), 7.70 (m, 1 H), 7.65 (d, J = 8.50 Hz, 1 H), 7.59 - 7.32 (m, 6 H), 4.86 (s, 1 H, N<u>CH</u>(Ph)CN), 4.83 (s, 2 H, O<u>$CH_2$</u>-Chinolin), 3.57 (m, 1 H, Chinolin-$CH_2$O<u>CH</u>($CH_2$-)$_2$), 2.87 (m, 1 H), 2.73 (m, 1 H), 2.51 (m, 1 H), 2.30 (m, 1 H), 2.02 - 1.91 (m, 2 H), 1.91 - 1.58 (m, 2 H).

**Beispiel XVII**

2-Cycloheptyl-2-(7-(chinolin-2-ylmethoxy)-naphth-2-yl)essigsäureethylester

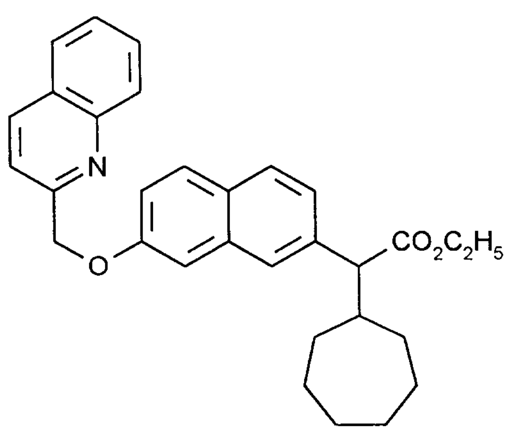

Natriumhydrid (0.132 g, 3.31 mmol, 60%ig in Paraffin) wird in wasserfreiem DMF (1 ml) unter Argon vorgelegt. Dazu werden 0.9 g (2.76 mmol) der Verbindung aus Beispiel III, gelöst in DMF (5 ml), bei 0°C zugetropft. Man läßt auf Raumtemperatur kommen und nach 30 Minuten wird wieder auf 0°C abgekühlt. Es werden (0.54 g = 3.03 mmol) 2-Chlormethylchinolin in DMF (4 ml) tropfenweise zugegeben. Es wird über Nacht bei Raumtemperatur gerührt. Die Lösung wird mit Wasser versetzt und 3 mal mit Ether extrahiert. Die vereinigten organischen Phasen werden über Natriumsulfat getrocknet und einrotiert. Der Rückstand wird säulenchromatographisch gereinigt:
Ausbeute: 1.21 g (94%);

$R_f$ = 0.73 (3:1; Petrolether : Essigester);
$R_f$ = 0.79 (50:1, $CH_2Cl_2$ : Methanol);
Masse (berechnet) für $C_{31}H_{33}NO_3$ = 467.61; Massenspektrum (CI ($NH_3$), rel. Intensität) 468 (80%), 144 (100%);
$^1H$ NMR (200 MHz, $CDCl_3$/TMS) δ 8.22 - 8.02 (m, 2 H), 7.88 - 7.66 (m, 5 H), 7.64 - 7.51 (m, 2 H), 7.36 (dd, J = 8.48 Hz, J = 1.70 Hz, 1 H), 7.31 - 7.19 (m, 2 H), 5.50 (s, 2 H), 4.10 (m, 2 H), 3.40 (d, J = 10.97 Hz, 1 H), 2.35 (m, 1 H), 1.90 - 1.20 (m, 11 H), 1.18 (t, J = 7.13 Hz, 3 H), 1.0 (m, 1 H).

**Beispiel XVIII**

2-Cyclopentyl-2-[6-(chinolin-2-ylmethoxy)naphth-2-yl]essigsäuremethylester

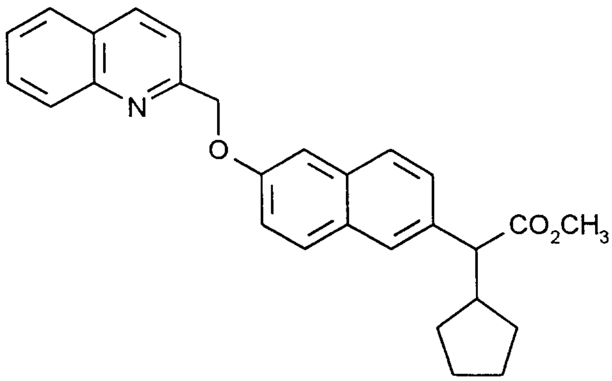

5.77 g (20.3 mmol) der Verbindung aus Beispiel VII und gemahlenes Kaliumcarbonat (5.61 g, 40.6 mmol) werden in DMF (50.0 ml) vorgelegt. Die Suspension wird auf 50°C für 1 Stunde erwärmt. Dazu gibt man vorsichtig festes 2-(Chlormethyl)chinolin hydrochlorid dazu. Es wird über Nacht bei 50°C nachgerührt. Man kühlt die Suspension ab und versetzt sie mit ca. 50 ml kaltem Wasser. Der entstandene Niederschlag wird abgesaugt und getrocknet:
Ausbeute: 7.81 g (91%);
Fp. = 124°C;
$R_f$ = 0.59 (50:1; $CH_2Cl_2$ : Methanol);
Masse (berechnet) für $C_{28}H_{27}NO_3$ = 425.53; Massenspektrum (EI, rel. Intensität) 425 (100%), 142 (80%);
$^1H$ NMR (200 MHz, $CDCl_3$): δ 8.17 (d, J = 8.57 Hz, 1 H), 8.10 (d, J= 7.67 Hz, 1 H), 7.90 - 7.50 (m, 6 H), 7.43 (dd, J = 8.52 Hz, J = 1.74 Hz, 1 H), 7.30 - 7.20 (m, 3 H), 5.50 (s, 2 H), 3.63 (s, 3 H), 3.39 (d, J = 11.12 Hz, 1 H), 2.65 (m, 1 H), 1.95 (m, 1 H), 1.70 - 1.20 (m, 6 H), 1.05 (m, 1 H).

**Beispiel XIX**

2-(5-(Chinolin-2-ylmethoxy)-pyridin-2-yl)-2-cycloheptylessigsäuremethylester

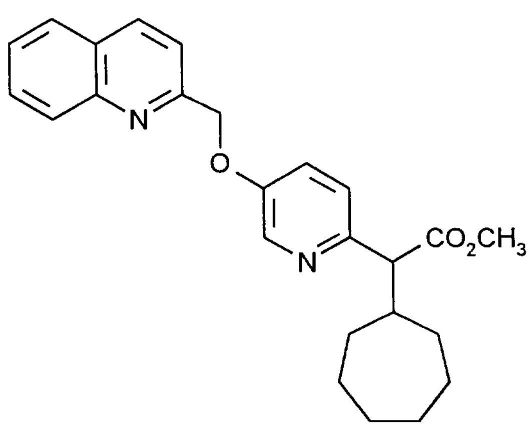

2-Chlormethylchinolin Hydrochlorid (6.07 g, 28.3 mmol), 7.0 g (28.3 mmol) der Verbindung aus Beispiel X und

gemahlenes Kaliumcarbonat (7.82 g, 56.6 mmol) werden über Nacht in DMF (100 ml) bei 40°C gerührt. Es wird abgekühlt und 150 ml Wasser zugetropft. Der entstandene Niederschlag wird abgesaugt und getrocknet. Die Kristalle werden aus Methanol umkristallisiert und getrocknet:
Ausbeute: 7.44 g (65%);
Fp. = 115°C;
$R_f$ = 0.21 (3:1; Petrolether : Essigester);
Masse (berechnet) für $C_{25}H_{28}N_2O_3$ = 404.51; Massenspektrum (FAB, rel. Intensität) 405 (100%), 345 (20%), 308 (20%);
$^1H$ NMR (250 MHz, $CDCl_3$/TMS) δ 8.39 (m, 1 H), 8.20 (d, J = 8.51 Hz, 1 H), 8.08 (d, J = 8.36 Hz, 1 H), 7.83 (dd, J = 8.14 Hz, J = 0.86 Hz, 1 H), 7.74 (m, 1 H), 7.65 (d, J = 8.51 Hz, 1 H), 7.30 (m, 1 H), 7.33 - 7.25 (m, 2 H), 5.39 (s, 2 H), 3.64 (s, 3 H), 3.60 (d, J = 10.63 Hz, 1 H), 2.38 (m, 1 H), 1.85 - 1.25 (m, 11 H), 1.08 (m, 1 H).

**Beispiel XX**

2-(5-(2-Chlorobenzyloxy)-pyridin-2-yl)-2-cycloheptylessigsäuremethylester

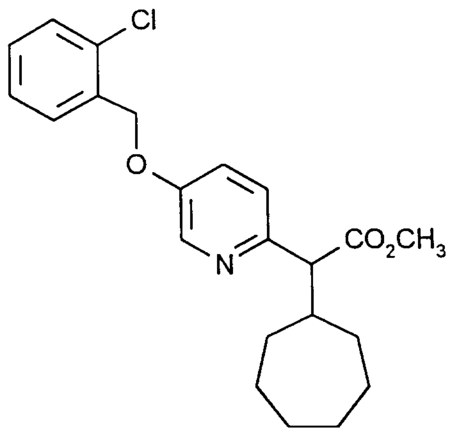

Natriumhydrid (1.0 g, 4.87 mmol, 60 % in Paraffin) wird in DMF (1 ml) vorgelegt und auf 0°C abgekühlt. 1.09 g (4.42 mmol) der Verbindung aus Beispiel X werden in DMF (9 ml) gelöst und zugetropft, die Temperatur steigt auf Raumtemperatur für 15 Minuten, anschließend wird wieder auf 0°C abgekühlt. Man tropft eine Lösung aus o-Chlorbenzylbromid (1.0 g, 4.87 mmol), gelöst in DMF (1 ml) hinzu. Man läßt auf Raumtemperatur erwärmen und nach 3 Stunden wird Wasser zugegeben und mit Diethylether 3x extrahiert. Die vereinigten organischen Phasen werden über $Na_2SO_4$ getrocknet und einrotiert. Der Rückstand wird säulenchromatographisch gereinigt;
Ausbeute: 1.43 g (83%);
$R_f$ = 0.59 (3:1; Petrolether : Essigester);
Masse (berechnet) für $C_{22}H_{26}ClNO_3$ = 387.91; Massenspektrum (EI, rel. Intensität) 387 (5%), 328 (15%), 291 (60%), 196 (40%), 181 (65%), 166 (100%);
$^1H$ NMR (200 MHz, $CDCl_3$/TMS) δ 8.33 (d, J = 2.79 Hz, 1 H), 7.53 (m, 1 H), 7.41 (m, 1 H), 7.34 - 7.20 (m, 4 H), 5.18 (s, 2 H), 3.65 (s, 3 H), 3.59 (d, J = 10.66 Hz, 1 H), 2.40 (m, 1 H), 1.90 - 1.35 (m, 11 H), 1.10 (m, 1 H).

**Beispiel XXI**

2-(5-(Chinolin-2-ylmethoxy)-pyridin-2-yl)-2-cyclopentylessigsäuremethylester

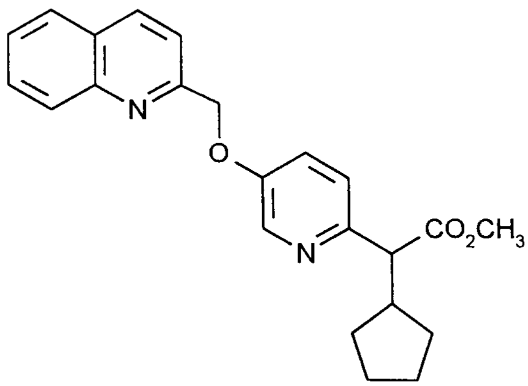

Die Reaktion wird mit 2-(Chlormethyl)chinolin Hydrochlorid (0.264 g, 1.23 mmol), gemahlenes Kaliumcarbonat (0.341 g, 2.46 mmol) und 0.290 g (1.23 mmol) der Verbindung aus Beispiel XI analog der Vorschrift des Beispiels XIX durchgeführt:
Ausbeute: 0.365 g (79%);
Fp. = 123°C;
$R_f$ = 0.14(50:1; $CH_2Cl_2$ : Methanol);
$R_f$ = 0.46 (20:1; $CH_2Cl_2$ : Methanol);
Masse (berechnet) für $C_{23}H_{24}N_2O_3$ = 376.46; Massenspektrum (FAB, rel. Intensität) 377 (100%);
$^1H$ NMR (250 MHz, $CDCl_3$/TMS) δ 8.38 (m, 1 H), 8.21 (d, J = 8.50 Hz, 1 H), 8.08 (d, J = 8.43 Hz, 1 H), 7.84 (dd, J = 8.17 Hz, J = 1.00 Hz, 1 H), 7.75 (m, 1 H), 7.65 (d, J = 8.52 Hz, 1 H), 7.56 (m, 1 H), 7.27 (m, 2 H), 5.39 (s, 2 H), 3.65 (s, 3 H), 3.54 (d, J = 11.05 Hz, 1 H), 2.65 (m, 1 H), 1.92 (m, 1 H), 1.70 - 1.20 (m, 6 H), 1.10 (m, 1 H).

**Beispiel XXII**

2-Cycloheptyl-2-[5-(chinolin-2-ylmethoxy)-thiophen-2-yl]essigsäureethylester

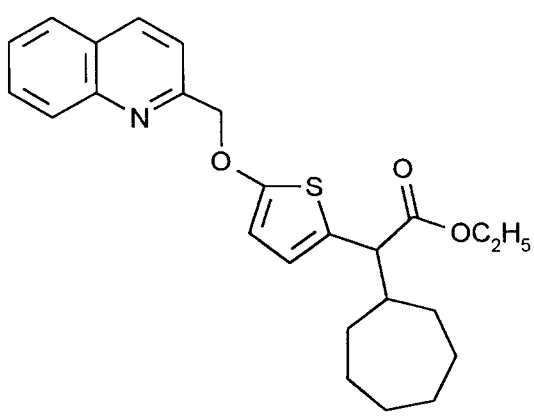

2-(Hydroxymethyl)chinolin (1.0 g, 6.3 mmol) wird in tert. Butanol (10 ml) gelöst und dazu gibt man langsam Natriumhydrid (0.25 g, 6.3 mmol). Es wird 30 Minuten auf 60°C gerührt und anschließend auf 100°C erwärmt. Man gibt 2.18 g (6.3 mmol) der Verbindung aus Beispiel XIII und Kupfer(I)bromid (0.09 g, 0.63 mmol) hinzu und läßt über Nacht bei 100°C nachrühren. Da wenig Umsetzung stattgefunden hat, wird weitere Kupfer(I)bromid zugegeben (0.81 g, 5.7 mmol). Nochmal wird 3 Stunden bei 100°C nachgerührt, abgekühlt, $CH_2Cl_2$ und gesättigte $NH_4Cl$-Lösung zugegeben und extrahiert. Die organische Phase wird über Natriumsulfat getrocknet und einrotiert. Der Rückstand wird säulenchromatographisch gereinigt:
Ausbeute: 0.10 g (2%);

$R_f$ = 0.25 (10:1; Petrolether : Essigester);
Masse (berechnet) für $C_{25}H_{29}NO_3S$ = 423.57; Massenspektrum (FAB, rel. Intensität) 424 (100%), 143 (90%).

**Beispiel XXIII**

N-(tert-Butoxycarbonyl)-4-(chinolin-2-ylmethoxy)piperidin

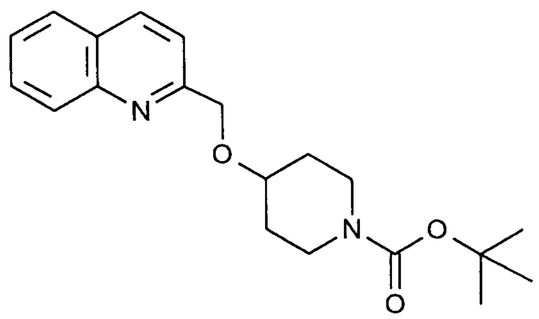

50.7 g (252 mmol) der Verbindung aus Beispiel XIV werden in DMF (500 ml) gelöst und auf 0°C abgekühlt. Es wird dazu portionsweise Natriumhydrid (31.7 g, 792 mmol, 60% in Paraffin) und Triethylamin (20 Tropfen) zugegeben. Es wird 2 Stunden bei Raumtemperatur nachgerührt, bis keine Gasentwicklung mehr zu beobachten ist. 2-(Chlormethyl)chinolin hydrochlorid (77.2 g, 360 mmol) wird in DMF (1000 ml) aufgeschlämmt und langsam zugegeben. Es wird über Nacht bei Raumtemperatur nachgerührt. Die Mischung wird auf Wasser (3000 ml) gegoßen und 2x mit Diethylether extrahiert. Die vereinigten organischen Phasen werden mit Wasser gewaschen, anschließend über $Na_2SO_4$ getrocknet und einrotiert. Der Rückstand wird Flashchromatographisch gereinigt:
Ausbeute: 90 g (>100%);
$R_f$ = 0.20 (3:1; Petrolether : Essigester);
Masse (berechnet) für $C_{20}H_{26}N_2O_3$ = 342.44; Massenspektrum (FAB, rel. Intensität) 342 (10%), 158 (100%), 143 (65%), 57 (100%);
$^1$H NMR (200 MHz, $CDCl_3$/TMS) δ 8.18 (d, J = 8.54 Hz, 1 H), 8.04 (d, J = 8.47 Hz, 1 H), 7.82 (dd, J = 8.17 Hz, J = 1.1 Hz, 1 H), 7.72 (m, 1 H), 7.65 (d, J = 8.53 Hz, 1 H), 7.54 (m, 1 H), 4.85 (s, 2 H, $O\underline{CH_2}$-Chinolin), 3.86 - 3.71 (m, 2 H, $N\underline{CH_2}CH_2$), 3.65 (m, 1 H, Chinolin-$CH_2O\underline{CH}(CH_2-)_2$, 3.17 - 3.04 (m, 2 H, $N\underline{CH_2}CH_2$), 1.97 - 1.85 (m, 2 H), 1.72 - 1.55 (m, 2 H), 1.46 (s, 9 H, $NCO_2C(CH_3)_3$).

**Beispiel XXIV**

2-Phenyl-[4-(chinolin-2-ylmethoxy)-piperidin-1-yl]-essigsäuremethylester

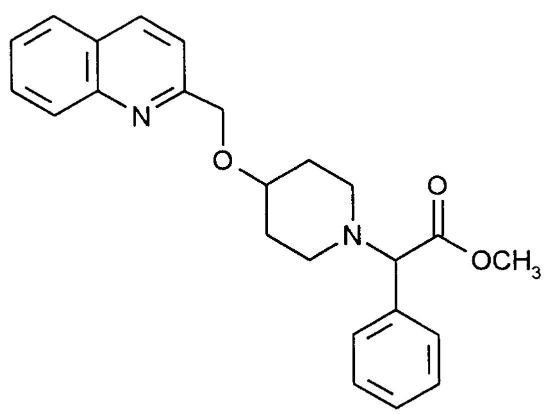

Zu einer auf 0°C abgekühlten Lösung aus 4.8 g (13.4 mmol) der Verbindung aus Beispiel XVI und Methanol (20 ml) wird vorsichtig Schwefelsäure (20 ml) zugetropft. Es wird über Nacht zum Rückfluß gekocht. Es wird abgekühlt und mit konz. Natronlauge auf pH ≈ 14 gestellt. Es wird mit Essigester extrahiert und die vereinigten organischen Phasen wer-

den über $Na_2SO_4$ getrocknet und einrotiert. Der Rückstand wird Flashchromatographisch gereinigt:
Ausbeute: 2.3 g (44%);
$R_f$ = 0.30 (1:1; Essigester : Petrolether);
Masse (berechnet) für $C_{24}H_{26}N_2O_3$ = 390.48; Massenspektrum (FAB, rel. Intensität) 391 (100%), 232 (40%);
$^1H$ NMR (250 MHz, $CDCl_3$/TMS) δ 8.15 (d, J = 8.49 Hz, 1 H), 8.02 (d, J = 8.28 Hz, 1 H), 7.81 (dd, J = 8.10 Hz, J = 1.0 Hz, 1 H), 7.69 (m, 1 H), 7.62 (d, J = 8.57 Hz, 1 H), 7.51 (m, 1 H), 7.44 - 7.38 (m, 2 H, Ph-H), 7.36 - 7.28 (m, 3 H, Ph-H), 4.81 (s, 2 H, O$CH_2$-Chinolin), 4.03 (s, 1 H, NCH(Ph)$CO_2CH_3$), 3.68 (s, 3 H, $CO_2CH_3$), 3.52 (m, 1 H, Chinolin-$CH_2O$CH($CH_2$-$)_2$), 2.80 - 2.68 (m, 2 H, N$CH_2CH_2$), 2.31 - 2.13 (m, 2 H, N$CH_2CH_2$), 2.05 - 1.89 (m, 2 H), 1.83- 1.70 (m, 2 H).

**Beispiel XXV**

2-[(5-Chinolin-2-yl-methoxy)-pyridin-2-yl]-2-cyclopentylessigsäure

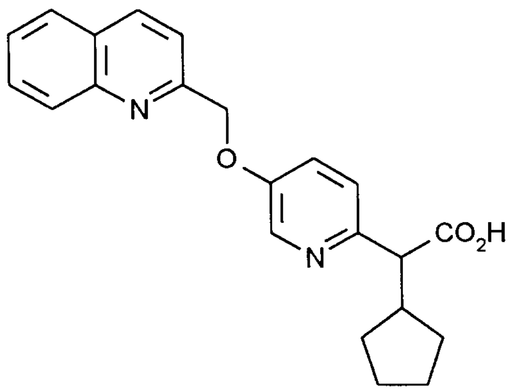

0.355 g (0.94 mmol) der Verbindung aus Beispiel XXI werden mit 2 N Natronlauge (1 ml) in Methanol (10 ml) über Nacht zum Rückfluß gekocht. Die Lösung wird abgekühlt und mit 2 N Salzsäure neutralisiert und abrotiert. Der Rückstand wird in $CH_2Cl_2$ gelöst und mit Wasser gewaschen. Die organische Phase wird über Natriumsulfat getrocknet und einrotiert. Der Rückstand wird säulenchromatographisch gereinigt (Kieselgel 60, Merck 40-63 µm, $CH_2Cl_2$ : Methanol, 100:10):
Ausbeute: 0.250 g (73% d.Th.);
$R_f$ = 0.36 ($CH_2Cl_2$ : Methanol, 100:10);
Fp.= 70°C (Schaum);
MS (FAB) 363 (100).

In Analogie zur Vorschrift des Beispiels XXV werden die in der Tabelle I aufgeführten Verbindungen hergestellt:

**Tabelle I:**

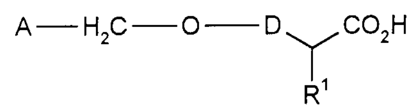

| Bsp.-Nr. | A | D | $R^1$ | Ausbeute (% d.Th.) | $R_f$ (Solvens) | MS (FAB) | Fp. (°C) | Ausgangsmaterial Bsp.-Nr. |
|---|---|---|---|---|---|---|---|---|
| XXVI | 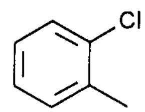 | 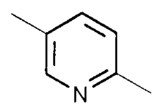 | (R&S) cHept | 90 | 0.34 (I) | 373 (80%), 231 (100%) | Schaum | XX |
| XXVII | 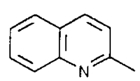 | 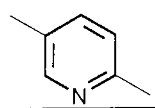 | (R&S) cHept | 21 | 0.31 (I) | 391 (20%), 176 (100%) | >235 | XIX |
| XXVIII | 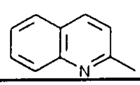 | 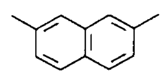 | (R&S) cHept | 89 | 0.11 (H) 0.59 (J) | -- | 112 | XVII |
| XXIX | 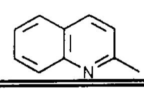 | 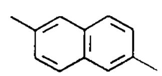 | (R&S) cPent | 86 | 0.14 (I) | 412 (100%), 154 (55%) | 234 | XVIII |

**Herstellungsbeispiele**

**Beispiel 1**

2-Cycloheptyl-N-(2-hydroxy-1(R)-phenylethyl)-2-[5-(chinolin-2-ylmethoxy)-thiophen-2-yl]essigsäureamid

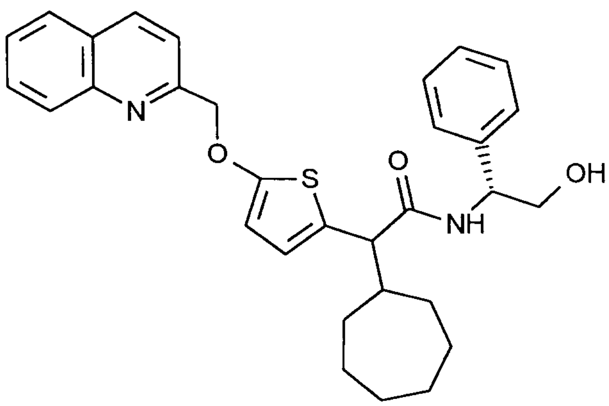

a)

Zu einer Lösung aus 0.1 g (0.23 mmol) der Verbindung aus Beispiel XXII und Ethanol (2.5 ml) wird 1 N Natronlauge (0.45 ml, 0.24 mmol) zugetropft. Die Lösung wird über Nacht zum Rückfluß gekocht. Es wird abgekühlt und mit 1N Salzsäure neutralisiert. Das Ethanol wird abrotiert, der Rückstand wird in Wasser und $CH_2Cl_2$ aufgenommen und extrahiert. Die vereinigten organischen Phasen werden über $Na_2SO_4$ getrocknet und einrotiert. Der Rückstand wird säulenchromatographisch gereinigt und anschließend nicht ganz rein weiter eingesetzt: Ausbeute: 0.02 g (22%)

b)

Eine Lösung aus R-Phenylglycinol (7 mg, 0.05 mmol) und $CH_2Cl_2$ (2 ml) wird vorgelegt. Dazu gibt man 20 mg (0.05 mmol) der unter a) aufgeführten Verbindung und 1-Hydroxybenzotriazol (7.5 mg, 0.056 mmol). Es wird auf 10 °C abgekühlt und anschließend mit N'-(3-Dimethylaminopropyl)-N'-ethylcarbodiimid (11 mg, 0.058 mmol) und Triethylamin (10.2 mg, 0.101 mmol) versetzt. Es wird über Nacht bei Raumtemperatur nachgerührt. Die Lösung wird ohne Aufarbeitung säulenchromatographisch gereinigt:

Ausbeute: 8.7 mg (33%);

$R_f$ = 0.46; ($CH_2Cl_2$ : Methanol, 20:1)

Masse (berechnet) für $C_{31}H_{34}N_2O_3S$ = 514.69, Massenspektrum (FAB, rel. Intensität) 515 (100%), 350 (25%), 154 (80%), 143 (80%), 136 (65%).

**Beispiel 2**

N-Benzyl-2-phenyl-2-[4-(chinolin-2-ylmethoxy)-piperidin-1-yl]acetamid

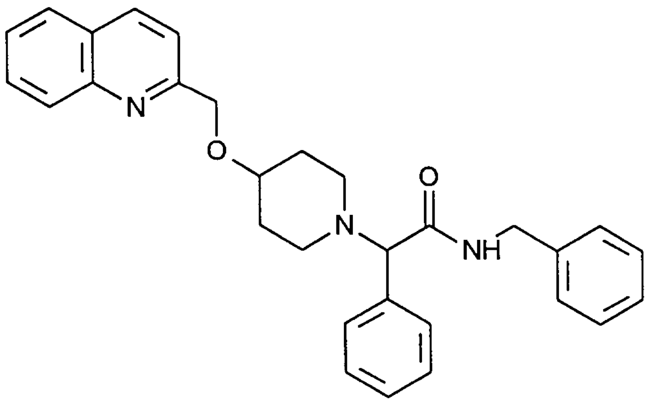

Es wird unter Argon Trimethylaluminium (1.53 ml, 3.07 mmol, 2 M in Hexan) in Toluol (10 ml) vorgelegt. Es wird auf 0°C abgekühlt, Benzylamin (0.301 g, 2.82 mmol) zugetropft und 1 Stunde bei Raumtemperatur nachgerührt. 1.0 g (2.56 mmol) der Verbindung aus Beispiel XXIV werden in Toluol (10 ml) gelöst und zugetropft. Es wird über Nacht bei 90°C nachgerührt. Man kühlt ab, gibt Wasser dazu und rührt kräftig für 10 Minuten nach. Es wird mit Essigester 2 x extrahiert, die vereinigten organischen Phasen werden über $Na_2SO_4$ getrocknet und einrotiert. Der Rückstand wird Flashchromatographisch gereinigt:
Ausbeute: 0.5 g (42%);
$R_f$ = 0.13; (1:1; Petrolether : EtOAc)
Masse (berechnet) für $C_{30}H_{31}N_3O_2$ = 465.60,
Massenspektrum (FAB, rel. Intensität) 466 (100%), 331 (90%), 91 (65%); $^1$H NMR (200 MHz, $CDCl_3$) $\delta$ 8.15 (d, J = 8.50 Hz, 1 H), 8.02 (d, J= 8.51 Hz, 1 H), 7.81 (dd, J = 8.11 Hz, J = 1.16 Hz, 1 H), 7.70 (m, 1 H), 7.59 (d, J = 8.49 Hz, 1 H), 7.58 - 7.40 (m, 2 H), 7.38 - 7.19 (m, 10 H), 4.78 (s, 2 H), 4.48 (d, J = 5.98 Hz, 2 H), 3.91 (s, 1 H), 3.49 (m, 1 H, Chinolin-$CH_2$-O$\underline{CH}$($CH_2$-$)_2$), 2.85 - 2.60 (m, 2 H, N$\underline{CH_2}$$CH_2$), 2.20 (m, 1 H), 2.10 - 1.80 (m, 3 H), 1.75 - 1.55 (m, 2 H).

In Analogie zur Vorschrift des Beispiels 1b werden die in der Tabelle 1 aufgeführten Verbindungen hergestellt:

Tabelle 1:

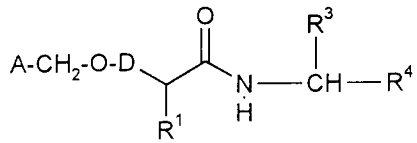

| Bsp.-Nr. | A | D | $R^1$ | $R^3$ | $R^4$ | Ausbeute (% d.Th.) | $R_f$ (Solvens) | MS (FAB, rel. Intens.) | Fp. (°C) | Ausgangs -material Bsp.-Nr. |
|---|---|---|---|---|---|---|---|---|---|---|
| 3 | 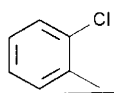 | 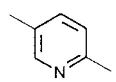 | (R&S) cHept | (R)-Phenyl | $-CH_2OH$ | 83 | 0.40 (I) | 493 (100%), 328 (80%) | 122 | XXVI |
| 4 | 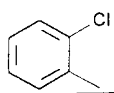 | 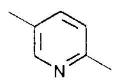 | (R&S) cHept | Phenyl | H | 49 | 0.56 (H) | 463 (100%), 328 (60%) | 124 | XXVI |
| 5 | 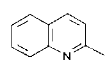 | 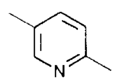 | (R&S) cPent | (R)-Phenyl | $-CH_2OH$ | 74 | 0.28 (I) | 482 (100%), 317 (80%) | 110 | XXV |
| 6 | 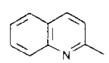 | 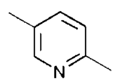 | (R&S) cHept | (R)-Phenyl | $-CH_2OH$ | 72 | 0.18 (I) | 510 (100%), 345(65%) | 58 | XXVII |
| 7 | 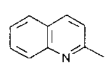 | 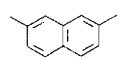 | (R&S) cHept | (R)-Phenyl | $-CH_2OH$ | 60 | 0.26 (H) | 559 (80%), 307 (100%) | - | XXVIII |

| Bsp.-Nr. | A | D | $R^1$ | $R^3$ | $R^4$ | Ausbeute (% d.Th.) | $R_f$ (Solvens) | MS (FAB, rel. Intens.) | Fp. (°C) | Ausgangs-material Bsp.-Nr. |
|---|---|---|---|---|---|---|---|---|---|---|
| 8 | 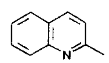 | 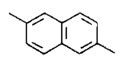 | (R&S) cPent | (R)-Phenyl | $-CH_2OH$ | 84 | 0.41 (I) | 531 (35%), 143 (100%) | 93 (Schaum) | XXIX |
| 9 | 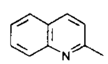 | 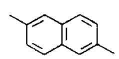 | (R&S) cPent | 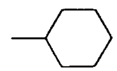 | H | 72 | 0.70 (I) | 507 (45%), 143 (100%) | 184 (Schaum) | XXIX |
| 10 | 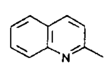 | 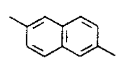 | (R&S) cPent | 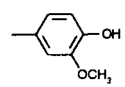 | H | 78 | 0.47 (I) | 547 (40%), 143 (90%), 137 (100%) | 108 (Schaum) | XXIX |
| 11 | 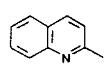 | 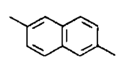 | (R&S) cPent | -(S)-Phenyl | $-CH_2OH$ | 63 | 0.35 (I) | 531 (30%), 143 (100%) | 100 (Schaum) | XXIX |

| Bsp.-Nr. | A | D | $R^1$ | $R^3$ | $R^4$ | Ausbeute (% d.Th.) | $R_f$ (Solvens) | MS (FAB, rel. Intens.) | Fp. (°C) | Ausgangs-material Bsp.-Nr. |
|---|---|---|---|---|---|---|---|---|---|---|
| 12 | 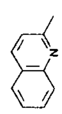 | 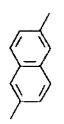 | (R&S) cPent | 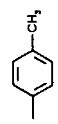 | H | 83 | 0.77 (I) | 515 (20%), 105 (100%) | 95 (Schaum) | XXIX |
| 13 | 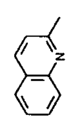 | 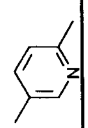 | (S) c Hept | (R)-Phenyl | $CH_2OH$ | | 0.51 (I) | | 140 | 6 |
| 14 | 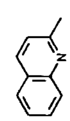 | 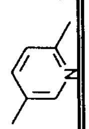 | (R) c Hept | (R)-Phenyl | $CH_2OH$ | | 0.50 (I) | | 150 | 6 |

## Patentansprüche

1. Heterocyclische Aryl-, Alkyl- und Cycloalkylessigsäureamide der allgemeinen Formel (I)

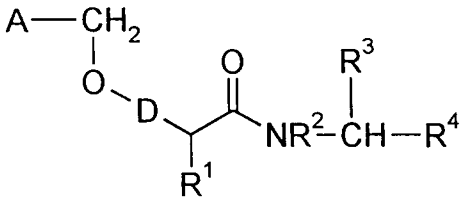

(I)

in welcher

A für Aryl mit 6 bis 10 Kohlenstoffatomen steht, oder für einen 5- bis 7-gliedrigen aromatischen, gegebenenfalls benzokondensierten Heterocyclus mit bis zu 3 Heteroatomen aus der Reihe S, N und/oder O steht, wobei die Ringe gegebenenfalls bis zu 3-fach gleich oder verschieden durch Halogen, Nitro, Cyano, Trifluormethyl, Hydroxy, Carboxyl oder durch geradkettiges oder verzweigtes Alkyl, Alkoxy, Acyl oder Alkoxycarbonyl mit jeweils bis zu 8 Kohlenstoffatomen substituiert sind,

D für Naphthyl, Cycloalkyl mit 3 bis 6 Kohlenstoffatomen oder für einen 4- bis 6-gliedrigen, gesättigten oder ungesättigten, gegebenenfalls benzokondensierten Heterocyclus mit bis zu 3 Heteroatomen aus der Reihe S, N und/oder O steht, wobei die Cyclen gegebenenfalls bis zu 2-fach gleich oder verschieden durch Halogen, Cyano, Hydroxy oder durch geradkettiges oder verzweigtes Alkyl oder Alkoxy mit jeweils bis zu 4 Kohlenstoffatomen substituiert sind,

$R^1$ für Wasserstoff, Cycloalkyl mit 3 bis 10 Kohlenstoffatomen oder für geradkettiges oder verzweigtes Alkyl mit 1 bis 10 Kohlenstoffatomen steht, oder
für Phenyl steht, das gegebenenfalls bis zu 2-fach gleich oder verschieden durch Halogen, Cyano, Hydroxy, geradkettiges oder verzweigtes Alkyl oder Alkoxy mit jeweils bis zu 4 Kohlenstoffatomen substituiert ist,

$R^2$ für Wasserstoff oder geradkettiges oder verzweigtes Alkyl mit bis zu 3 Kohlenstoffatomen steht,

$R^3$ für Wasserstoff oder geradkettiges oder verzweigtes Alkyl mit bis zu 5 Kohlenstoffatomen oder Benzyl steht, oder
für Cycloalkyl mit 3 bis 7 Kohlenstoffatomen steht, oder
für Phenyl oder für einen 5- bis 7-gliedrigen aromatischen Heterocyclus mit bis zu 3 Heteroatomen aus der Reihe S, N und/oder O steht, die gegebenenfalls bis zu 3-fach gleich oder verschieden durch Halogen, Nitro, Phenyl, Hydroxy oder durch geradkettiges oder verzweigtes Alkyl oder Alkoxy mit bis zu 6 Kohlenstoffatomen substituiert sind,

und

$R^4$ für Wasserstoff oder für eine Gruppe der Formel $-CH_2-OH$ oder $CH_2O-CO-R^5$ steht,
worin

$R^5$ Wasserstoff, geradkettiges oder verzweigtes Alkyl mit bis zu 8 Kohlenstoffatomen oder Phenyl bedeutet, das gegebenenfalls bis zu 3-fach gleich oder verschieden durch Halogen, Hydroxy, Cyano oder geradkettiges oder verzweigtes Alkyl oder Alkoxy mit jeweils bis zu 4 Kohlenstoffatomen substituiert ist,

und deren Salze.

2. Heterocyclische Aryl-, Alkyl- und Cycloalkylessigsäureamide der Formel nach Anspruch 1
in welcher

A für Phenyl, Chinolyl, Indolyl, Isochinolyl, Benzo[b]furyl, Benzo[b]thienyl oder Pyridyl steht, die gegebenenfalls bis zu 2-fach gleich oder verschieden durch Fluor, Chlor, Brom, Nitro, Cyano, Trifluormethyl, Hydroxy, Carboxyl oder durch geradkettiges oder verzweigtes Alkyl, Alkoxy, Acyl oder Alkoxycarbonyl mit jeweils bis zu 6 Kohlenstoffatomen substituiert sind,

D für Naphthyl, Cyclobutyl, Cyclopentyl, Cyclohexyl, Pyridyl, Furyl, Thienyl, Piperidinyl, Pyrimidinyl, Piperazinyl, Pyridazinyl oder Azetidinyl steht, die gegebenenfalls durch Fluor, Chlor, Brom, Cyano, Hydroxy, Methyl oder Methoxy substituiert sind,

$R^1$ für Wasserstoff, Cyclobutyl, Cyclopentyl, Cyclohexyl, Cycloheptyl, Cylcooctyl oder für geradkettiges oder verzweigtes Alkyl mit bis zu 7 Kohlenstoffatomen steht, oder
für Phenyl steht, das gegebenenfalls durch Fluor, Chlor, Brom, Cyano, Hydroxy oder geradkettiges oder verzweigtes Alkyl oder Alkoxy mit jeweils bis zu 3 Kohlenstoffatomen substituiert ist,

$R^2$ für Wasserstoff oder Methyl steht,

$R^3$ für Wasserstoff oder geradkettiges oder verzweigtes Alkyl mit bis zu 4 Kohlenstoffatomen, Benzyl, Cyclopropyl, Cyclopentyl, Cyclohexyl oder für Phenyl, Pyridyl, Thienyl oder Furyl steht, die gegebenenfalls bis zu 2-fach gleich oder verschieden durch Fluor, Chlor, Brom, Phenyl, Nitro, Hydroxy oder durch geradkettiges oder verzweigtes Alkyl oder Alkoxy mit bis zu 4 Kohlenstoffatomen substituiert sind,

und

$R^4$ für Wasserstoff oder für eine Gruppe der Formel $-CH_2-OH$ oder $-CH_2O-CO-R^5$ steht,
worin

$R^5$ Wasserstoff, geradkettiges oder verzweigtes Alkyl mit bis zu 6 Kohlenstoffatomen oder Phenyl bedeutet, das gegebenenfalls bis zu 2-fach gleich oder verschieden durch Fluor, Chlor, Brom, Cyano, Hydroxy oder geradkettiges oder verzweigtes Alkyl oder Alkoxy mit jeweils bis zu 3 Kohlenstoffatomen substituiert ist,

und deren Salze.

**3.** Heterocyclische Aryl-, Alkyl- und Cycloalkylessigsäureamide der Formel nach Anspruch 1
in welcher

A für Phenyl, Chinolyl oder Pyridyl steht, die gegebenenfalls durch Fluor, Chlor, Brom, Cyano oder durch geradkettiges oder verzweigtes Alkyl, Alkoxy, Acyl oder Alkoxycarbonyl mit jeweils bis zu 3 Kohlenstoffatomen substituiert sind,

D für Naphthyl, Cyclobutyl, Cyclopentyl, Cyclohexyl, Furyl, Pyridyl, Thienyl, Azetidinyl oder Piperidinyl steht, die gegebenenfalls durch Fluor, Cyano, Hydroxy, Methyl oder Methoxy substituiert sind,

$R^1$ für Wasserstoff, Cyclohexyl, Cycloheptyl, Cyclooctyl oder für geradkettiges oder verzweigtes Alkyl mit bis zu 6 Kohlenstoffatomen steht, oder
für Phenyl steht, das gegebenenfalls durch Fluor, Chlor, Cyano, Hydroxy, Methyl oder Methoxy substituiert ist,

$R^2$ für Wasserstoff oder Methyl steht,

$R^3$ für Wasserstoff oder geradkettiges oder verzweigtes Alkyl mit bis zu 3 Kohlenstoffatomen, Benzyl, Cyclopropyl, Cyclopentyl, Cyclohexyl oder für Pyridyl, Thienyl oder Phenyl steht, die gegebenenfalls bis zu 2-fach gleich oder verschieden durch Fluor, Chlor, Brom, Nitro, Phenyl, Hydroxy oder durch geradkettiges oder verzweigtes Alkyl oder Alkoxy mit bis zu 3 Kohlenstoffatomen substituiert sind,

und

$R^4$ für Wasserstoff, für eine Gruppe der Formel $-CH_2-OH$ oder $-CH_2O-CO-R^5$ steht,
worin

$R^5$ geradkettiges oder verzweigtes Alkyl mit bis zu 6 Kohlenstoffatomen oder Phenyl bedeutet, das gegebenenfalls bis zu 2-fach gleich oder verschieden durch Fluor, Chlor, Brom, Cyano, Hydroxy, Methyl oder Methoxy substituiert ist,

und deren Salze.

4. Heterocyclische Aryl-, Alkyl- und Cycloalkylessigsäureamide nach Anspruch 1 bis 3 zur therapeutischen Anwendung.

5. Verfahren zur Herstellung von Heterocyclischen Aryl-, Alkyl- und Cycloalkylessigsäureamiden nach Anspruch 1 bis 3, dadurch gekennzeichnet, daß man Carbonsäuren oder Ester der allgemeinen Formel (II)

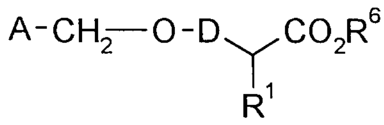

(II)

in welcher

A, D und $R^1$ die oben angegebene Bedeutung haben,

und

$R^6$ für Wasserstoff oder für geradkettiges oder verzweigtes Alkyl mit bis zu 4 Kohlenstoffatomen steht,

mit Aminen der allgemeinen Formel (III)

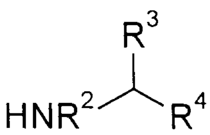

(III)

in welcher

$R^2$, $R^3$ und $R^4$ die oben angegebene Bedeutung haben,

gegebenenfalls in komplexierter Form in inerten Lösemitteln und in Anwesenheit von Basen und/oder Hilfsstoffen umsetzt.

6. Arzneimittel enthaltend mindestens 1 heterocyclisches Aryl-, Alkyl-oder Cycloalkylessigsäureamid nach Anspruch 1 bis 3.

7. Arzneimittel nach Anspruch 6 zur Behandlung von Atherosklerose.

8. Verfahren zur Herstellung von Arzneimitteln nach Anspruch 6 und 7, dadurch gekennzeichnet, daß man den Wirkstoff gegebenenfalls mit üblichen Hilfs- und Trägerstoffen in eine geeignete Applikationsform überführt.

9. Verwendung von heterocyclischen Aryl-, Alkyl- und Cycloalkylessigsäureamiden nach Anspruch 1 bis 3 zur Herstellung von Arzneimitteln.

10. Verwendung nach Anspruch 9 zur Herstellung von antiatherosklerotischen Arzneimitteln.

Europäisches Patentamt

# EUROPÄISCHER TEILRECHERCHENBERICHT

der nach Regel 45 des Europäischen Patent-übereinkommens für das weitere Verfahren als europäischer Recherchenbericht gilt

Nummer der Anmeldung
EP 96 11 4868

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.6) |
|---|---|---|---|
| A | EP 0 545 170 A (BAYER AG) 9.Juni 1993<br>* das ganze Dokument *<br>--- | 1-10 | C07D409/12<br>C07D401/12<br>C07D215/14<br>C07D213/65<br>A61K31/44<br>A61K31/47 |
| X | US 4 248 788 A (BOURGERY GUY R ET AL) 3.Februar 1981<br>* Spalte 24 - Spalte 25 *<br>--- | 1 | |
| X | CH 380 129 A (SANDOZ LTD) 15.September 1964<br>* Beispiele 2,3 *<br>--- | 1 | |
| X | ANNALES PHARMACEUTIQUES FRANCAISES, Bd. 36, Nr. 9-10, 1978, PARIS FR, Seiten 401-408, XP002022243<br>J.C. GAIGNAULT ET AL.: "Indolylméthyl-1 têtrahydro-1,2,3,4-isoquinoléines et benzyl-1 têtrahydro-2,3,4,9/1H/beta-carbolines formées à partir de la tryptamine et de ses dérivés"<br>* Abbildung 2 *<br>---<br>-/-- | 1 | RECHERCHIERTE SACHGEBIETE (Int.Cl.6)<br>C07D<br>A61K |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.
Vollständig recherchierte Patentansprüche:
Unvollständig recherchierte Patentansprüche:
Nicht recherchierte Patentansprüche:
Grund für die Beschränkung der Recherche:

Siehe Ergänzungsblatt C

| Recherchenort | Abschlußdatum der Recherche | Prüfer |
|---|---|---|
| DEN HAAG | 8.Januar 1997 | Bosma, P |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C09)

Europäisches Patentamt

# EUROPÄISCHER TEILRECHERCHENBERICHT

Nummer der Anmeldung
EP 96 11 4868

| EINSCHLÄGIGE DOKUMENTE | | | KLASSIFIKATION DER ANMELDUNG (Int.Cl.6) |
|---|---|---|---|
| Kategorie | Kennzeichnung des Dokuments mit Angabe, soweit erforderlich der maßgeblichen Teile | Betrifft Anspruch | |
| X | HELVETICA CHIMICA ACTA,<br>Bd. 49, Nr. 3, 1966, BASEL CH,<br>Seiten 1199-1203, XP002022244<br>R. STAUFFER: "Synthèse de nouvelles tryptamines substituées "<br>*Verbindung V*<br>----- | 1 | |
| | | | RECHERCHIERTE SACHGEBIETE (Int.Cl.6) |

Europäisches Patentamt

EP 96 11 4868 - C -

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 2,3
Unvollständig recherchierte Patentansprüche: 1,4-10
Nicht recherchierte Patentansprüche:

Grund für die Beschränkung der Recherche: Der Gegenstand der Ansprüche umfasst einen zu grossen Bereich von chemisch grundverschiedenen Resten. Daher ist eine vollständige Recherche aus ökonomischen Gründe nicht möglich und ist der Recherchenbericht nicht als vollständig anzusehen. (Siehe Richtlinien für die Europaischen Patentamt, Teil B, Kapitel III, 2). In Anlehnung an den Geist und das erfinderische Konzept der vorliegenden Anmeldung ist die Recherche auf die Beispiele und die angegebenen Ansprüche beschränkt worden.

EPO Form Supplementary Sheet C (1996)